(19) Europäisches Patentamt
European Patent Office
Office européen des brevets

(11) **EP 3 193 828 B1**

(12) **EUROPEAN PATENT SPECIFICATION**

(45) Date of publication and mention of the grant of the patent:
**01.03.2023 Bulletin 2023/09**

(21) Application number: **15762884.3**

(22) Date of filing: **28.08.2015**

(51) International Patent Classification (IPC):
**A61K 9/00** (2006.01)    **A61K 47/10** (2006.01)
**A61K 47/26** (2006.01)    **A61K 47/36** (2006.01)
**A61K 39/00** (2006.01)    **A61M 37/00** (2006.01)
**A61B 17/20** (2006.01)    **B29C 43/02** (2006.01)

(52) Cooperative Patent Classification (CPC):
**A61M 37/0015; A61K 9/0021; A61K 39/00;**
**B29C 33/424; B29C 39/003; B29C 39/025;**
**B29C 39/10; B29C 39/40;** A61M 2037/0023;
A61M 2037/0046; A61M 2037/0053;
B29C 2043/026; B29K 2105/0035; B29K 2883/00;
B29L 2031/7544;                              (Cont.)

(86) International application number:
**PCT/US2015/047563**

(87) International publication number:
**WO 2016/033540 (03.03.2016 Gazette 2016/09)**

(54) **MICROSTRUCTURE ARRAY FOR DELIVERY OF ACTIVE AGENTS**

MIKROSTRUKTURANORDNUNG ZUR FREISETZUNG VON WIRKSTOFFEN

RÉSEAUX À MICROSTRUCTURES POUR L'ADMINISTRATION D'AGENTS ACTIFS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.08.2014  US 201462044051 P**

(43) Date of publication of application:
**26.07.2017  Bulletin 2017/30**

(73) Proprietor: **Corium, Inc.**
**Menlo Park, CA 94025 (US)**

(72) Inventors:
• **CHEN, Guohua**
**Sunnyvale, California 94086 (US)**
• **KATIKANENI, Sahitya**
**Santa Clara, California 95050 (US)**
• **GHARTEY-TAGOE, Esi**
**San Jose, CA 95134 (US)**
• **SINGH, Parminder**
**Union City, California 94587 (US)**

(74) Representative: **D Young & Co LLP**
**120 Holborn**
**London EC1N 2DY (GB)**

(56) References cited:
**WO-A1-2007/061964    WO-A1-2014/144973**
**WO-A1-2014/150069    WO-A1-2014/151654**
**WO-A2-2005/099751    US-A1- 2006 067 943**
**US-A1- 2009 035 446    US-A1- 2009 041 810**
**US-A1- 2009 155 330    US-A1- 2011 098 651**

• **LEI GUO ET AL: "Enhanced transcutaneous**
**immunization via dissolving microneedle array**
**loaded with liposome encapsulated antigen and**
**adjuvant", INTERNATIONAL JOURNAL OF**
**PHARMACEUTICS, ELSEVIER BV, NL, vol. 447,**
**no. 1-2, 15 April 2013 (2013-04-15), pages 22-30,**
**XP002715005, ISSN: 0378-5173, DOI:**
**10.1016/J.IJPHARM.2013.02.006 [retrieved on**
**2013-02-11]**

**(Cont. next page)**

- **HOLLY J. CORBETT ET AL: "Skin Vaccination against Cervical Cancer Associated Human Papillomavirus with a Novel Micro-Projection Array in a Mouse Model", PLOS ONE, vol. 5, no. 10, 18 October 2010 (2010-10-18), pages 1-9, XP055226369, US ISSN: 1932-6203, DOI: 10.1371/journal.pone.0013460**
- **DATABASE WPI Section Ch, Week 201480 Thomson Scientific, London, GB; Class A96, AN 2014-V89218 XP002750688, GAO Y; GUO L: "Soluble microneedle patch useful for transdermal administration of vaccine, comprises water-soluble polymer material as matrix material and soluble microneedle main portion", -& CN 104 027 324 A (TECH INST PHYSICS & CHEM CHINESE ACAD) 10 September 2014 (2014-09-10)**

(52) Cooperative Patent Classification (CPC): (Cont.)
B29L 2031/756

**Description**

CROSS-REFERENCE TO RELATED APPLICATIONS

This application claims the benefit of U.S. Provisional Application No. 62/044,051, filed August 29, 2014.

TECHNICAL FIELD

The disclosure relates generally to a method of making a microstructure array comprising a vaccine active agent, and to a microstructure array obtained by this method.

BACKGROUND

Arrays of microneedles were proposed as a way of administering drugs through the skin in the 1970s. Microneedle or microstructure arrays can facilitate the passage of drugs through or into human skin and other biological membranes in circumstances where ordinary transdermal administration is inadequate. Microstructure arrays can also be used to sample fluids found in the vicinity of a biological membrane such as interstitial fluid, which is then tested for the presence of biomarkers.

In recent years, microstructure arrays have been prepared in a manner that makes financially feasible their widespread use. U.S. Pat. No. 6,451,240 discloses illustrative methods of manufacturing microneedle arrays. If the arrays are sufficiently inexpensive, they can be provided as disposable devices. A disposable device is preferable to a reusable device since the integrity of the device is not compromised due to prior use, and the potential need of resterilizing the device after each use and maintaining the device under controlled storage conditions is eliminated. Moreover, microstructure arrays are advantageous for use in developing countries, since the need for needles and refrigeration is eliminated.

Despite much initial work on fabricating microneedle arrays using silicon or metals, there are significant advantages to polymeric rather than metal or silicon-based arrays. Methods of manufacturing polymeric microneedle arrays are described in U.S. Patent No. 6,451,240, while arrays prepared primarily of biodegradable polymers have also been described. See, e.g. U.S. Pat. No. 6,945,952 and U.S. Published Patent Application Nos. 2002/0082543 and 2005/0197308. A detailed description of the fabrication of an illustrative microneedle array made of polyglycolic acid (PGA) is found in Park et al., J. of Controlled Release, 104:51-66 (2005). Vaccine delivery via microneedle arrays is described, for example, in U.S. Patent Publication No. 2009/0155330. Dissolving microprojection arrays are also described therein.

Transdermal delivery of vaccines using microneedles have recently been described including coating or encapsulating vaccine onto or within microneedles (Prausnitz et al., Curr Top Microbiol Immunol, 2009, 333:369-393). Intradermal administration elicited the same immune responses at lower doses as compared to intramuscular injection. Prausnitz *et al.* makes no mention of using an adjuvant in the vaccine formulation.

Insoluble aluminum salts have been used for nearly 80 years as immunologic adjuvants. US 2009/035446 A1 discloses a method of producing a microneedle with a selected drug concentrated in the tip or on the tip surface, wherein the method comprises: (1) providing a particulate component comprising an inert particle with an adsorbed drug, wherein the drug is a vaccine or a protein; (2) combining the particulate component with a soluble matrix material to form a suspension/solution comprising the particulate component; (3) casting the suspension/solution into a microneedle mold; (4) centrifuging under conditions that move the particulate component into the microneedle tip or tip surface; and (5) drying and separating the microneedle from the mold. The inert particle is poly (lactic-co-glycolic acid), aluminum hydroxide or aluminum phosphate. The matrix material is a hydrogel, preferably comprising sodium carboxymethyl cellulose. The use of aluminum salts as an adjuvant is known to produce long-lived subcutaneous nodules. It was long presumed that these nodules were central for adjuvant activity by providing a depot of the antigen. A recent study concluded that the formation of nodules is not required for the ability of aluminum salts to act as a depot to retain antigen in the body or to act as adjuvants (Munks, etal., Blood, 2010, 116(24):5191-5199).

Microneedle-assisted transdermal delivery of therapeutic agents is a fairly recent technological development. There exists a current need for improved formulations and microprojection arrays for effectively delivering active agents, via the skin, while providing good formulation stability (including maintenance of active agent potency) upon manufacturing and storage, and during administration, to thereby conveniently deliver a therapeutically and/or immunogenically effective amount of active agent without the associated discomfort, inconvenience, or chemical instability of traditional liquid-based, needle-based methods.

BRIEF SUMMARY

The invention is defined by the claims. Any subject-matter falling outside the scope of the claims is provided for information purposes only.

In a first aspect, the present invention concerns a method of making a microstructure array, comprising: (i) providing an aqueous liquid formulation comprising a vaccine active agent, an insoluble particulate aluminum salt adjuvant, a hydrophilic polymer selected from the group consisting of polysaccharides, polyvinylpyrrolidones, polyvinyl alcohol, polyethylene glycol, copolymers of ethylene glycol and propylene glycol, and block copolymers of poly(lactic acid-co-glycolic acid) and polyethylene glycol, and at least one co-solvent selected from the group consisting of isopropyl alcohol, ethanol, methanol and butanol in an aqueous buffer; (ii) dispensing the liquid formulation from step (i) onto a mold having an array of microstructure cavities and filling the microstructure cavities to form a formulation-filled mold; (iii) removing excess liquid formulation from a top surface of the mold; (iv) drying the formulation-filled mold; (v) placing a backing layer on the dried mold from (iv), whereby the backing layer forms a base having an attachment point to the formulation dried in each of the microstructure cavities to provide a molded microstructure array; and (vi) removing the microstructure array from (v) from the mold. In a further embodiment, the mold is purged with a soluble gas prior to dispensing the formulation onto the mold. In specific embodiments, the soluble gas is selected from $CO_2$ and $CH_4$. In other embodiments, the method includes applying pressure to the formulation and mold after dispensing the liquid formulation on the mold. In an embodiment, the pressure is applied after excess formulation is removed from the mold surface. In one embodiment, pressure of at least about 68.95-206.84 kPa (10-30 psi) above atmospheric is applied. In another embodiment, pressure is applied for at least about 5 seconds to at least about 2 minutes.

In an embodiment, the above method comprises drying the formulation filled mold at about 5-50° C for about 20 minutes to about two hours. In further embodiments, the formulation filled mold is dried for about 30-60 minutes. In specific embodiments, the formulation filled mold is dried for at least about 20 minutes, 30 minutes, 45 minutes, 60 minutes, 90 minutes, or two hours. In other embodiments, the method includes drying the backing layer formulation. In embodiments, drying the backing layer formulation comprises drying the mold and/or array in an oven at about 5-50° C.

In another embodiment related to the above, the above method further comprises affixing a backing substrate to a backing layer. Exemplary backing substrates include, e.g., a pressure sensitive adhesive, a breathable non-woven impregnated pressure sensitive adhesive, and an ultraviolet-cured adhesive in a polymer (e.g. polycarbonate or polyester) film.

In yet another embodiment, the method includes an additional drying step including drying the microstructure array 5-50° C for at least about 2-12 hours. In embodiments, the additional drying step is performed at about 35° C. In other embodiments, the drying is performed under vacuum. In specific embodiments, the drying is performed in a chamber having a partial pressure of water of about 6.66 Pa (0.05 Torr).

In further embodiments, the liquid formulation further comprises at least one of a sugar, a surfactant, or an antioxidant. In additional embodiments, the sugar is selected from sorbitol, sucrose, trehalose, fructose, or dextrose. In other embodiments, the surfactant is selected from Polysorbate 20 or Polysorbate 80. In yet other embodiments, the antioxidant is selected from methionine, cysteine, D-alpha tocopherol acetate, EDTA, or vitamin E. In additional embodiments, the liquid formulation is a solution or a suspension.

In a second aspect, the invention provides a microstructure array obtained by the method described above, comprising: an approximately planar base having a first surface and a second surface opposed thereto; a plurality of biodegradable microstructures extending outwardly from the base, each microstructure having an attachment point to the base and a distal portion to penetrate a subject's skin, wherein (i) the entire distal portion of each microstructure in the plurality of microstructures comprises at least one vaccine antigen active agent, and an insoluble particulate aluminum salt adjuvant in a biocompatible and water-soluble polymer matrix, the biocompatible and water-soluble polymer matrix comprising at least one structure forming polymer, wherein said structure forming polymer is a hydrophilic polymer selected from polysaccharides, polyvinyl pyrrolidone, polyvinyl alcohol, polyethylene glycol, copolymers of ethylene glycol and propylene glycol and block copolymers of poly(lactic acid-co-glycolic acid) and polyethylene glycol; and (ii) the base comprises a biocompatible non-water soluble polymer matrix, wherein the microstructures, upon penetration of the subject's skin, undergo dissolution to thereby deliver the antigen active agent and the particulate aluminum salt adjuvant. In a further embodiment, the vaccine antigen active agent is directed against at least one of adenovirus, anthrax, diphtheria, hepatitis, Haemophilus influenza a and/or b, human papillomavirus, influenza, Japanese encephalitis, Lyme disease, measles, meningococcal and pneumococcus infection, mumps, pertussis, polio, rabies, rotavirus, rubella, shingles, smallpox, tetanus, tuberculosis, typhoid, varicella, or yellow fever.

In particular embodiments, the aluminum salt is selected from aluminum hydroxide, aluminum potassium sulfate, and aluminum phosphate.

In further embodiments, the matrix further comprises one or more excipients. In some embodiments, the microstructures further comprise at least one of a sugar, a surfactant, or an antioxidant. In particular embodiments, the at least one sugar is selected from sorbitol, sucrose, trehalose, fructose, and dextrose. In other particular embodiments, the surfactant is selected from Polysorbate 20 or Polysorbate 80. In yet further embodiments, the antioxidant is selected from methionine, cysteine, D-alpha tocopherol acetate, EDTA, or vitamin E.

In additional embodiments, the microstructure array further comprises a backing substrate affixed to the planar base on an opposite side from the plurality of microstructures.

In embodiments, the microstructures have a diamond cross-section. In further embodiments, the microstructures have a height from tip to the backing layer of at least about 50-500 $\mu$m. In other embodiments, the microstructures have a height of about 100-300 $\mu$m. In yet other embodiments, the microstructures have a height of at least about 200 $\mu$m.

Provided herein but not according to the invention is a method of administering a vaccine or immunizing a subject. The method comprises applying a microstructure array as described herein to the subject, wherein formation of granulomas in the skin is reduced as compared to other forms of administering a vaccine, including subcutaneous or intradermal administration.

Additional embodiments of the present microstructures, arrays, methods, and the like, will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent.

Additional aspects and advantages of the present invention are set forth in the following description and claims, particularly when considered in conjunction with the accompanying examples and drawings.

## BRIEF DESCRIPTION OF DRAWINGS

FIGS. 1A-1 B are images of microstructure arrays prepared with (FIG. 1A) and without pressurization (FIG. 1B).

FIGS. 2A-2B are images of dried formulations in the mold prepared with (FIG. 2A) and without pressurization (FIG. 2B).

FIG. 3 is an illustration of an exemplary microstructure array.

FIG. 4 is an illustration of an exemplary method of forming a microstructure array.

FIGS. 5A-5E are illustrations of exemplary shapes for microstructures of the arrays described herein.

## DETAILED DESCRIPTION

Various aspects of the microstructure array, active agent formulations, and related methods will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

The practice of the present disclosure will employ, unless otherwise indicated, conventional methods of chemistry, biochemistry, and pharmacology, within the skill of the art. Such techniques are explained fully in the literature. See, *e.g.;* A.L. Lehninger, Biochemistry (Worth Publishers, Inc., current addition); Morrison and Boyd, Organic Chemistry (Allyn and Bacon, Inc., current addition); J. March, Advanced Organic Chemistry (McGraw Hill, current addition); Remington: The Science and Practice of Pharmacy, A. Gennaro, Ed., 20th Ed.; Goodman & Gilman The Pharmacological Basis of Therapeutics, J. Griffith Hardman, L. L. Limbird, A. Gilman, 10th Ed.

Where a range of values is provided, it is intended that each intervening value between the upper and lower limit of that range and any other stated or intervening value in that stated range is encompassed within the disclosure. For example, if a range of 1 $\mu$m to 8 $\mu$m is stated, it is intended that 2 $\mu$m, 3 $\mu$m, 4 $\mu$m, 5 $\mu$m, 6 $\mu$m, and 7 $\mu$m are also explicitly disclosed, as well as the range of values greater than or equal to 1 $\mu$m and the range of values less than or equal to 8 $\mu$m.

## Definitions

As used in this specification, the singular forms "a," "an," and "the" include plural referents unless the context clearly dictates otherwise. Thus, for example, reference to a "polymer" includes a single polymer as well as two or more of the same or different polymers, reference to an "excipient" includes a single excipient as well as two or more of the same or different excipients, and the like.

In describing and claiming the present invention, the following terminology will be used in accordance with the definitions described below.

An "adjuvant" as used herein refers generally to an agent that modifies the effect of other agents. In a particular use, "adjuvant" refers to an agent included in a vaccine formulation to modify the immune response of the vaccine.

"Biodegradable" refers to natural or synthetic materials that degrade enzymatically, non-enzymatically or both to produce biocompatible and/or toxicologically safe by-products which may be eliminated by normal metabolic pathways.

"Hydrophobic polymer" as used herein refers to polymers that are insoluble or poorly soluble in aqueous solvents. "Hydrophilic polymer" as used herein refers to polymers that are soluble or substantially soluble in aqueous solvents.

The terms "microprotrusion", "microprojection", "microstructure" or "microneedle" are used interchangeably herein to refer to elements adapted to penetrate or pierce at least a portion of the stratum corneum or other biological membranes. For example, illustrative microstructures may include, in addition to those described herein, microblades as described in U.S. Patent No. 6,219,574, edged microneedles as described in U.S. Patent No. 6,652,478, and microprotrusions as described in U.S. Patent Publication No. U.S. 2008/0269685 and U.S. 2009/0155330.

"Optional" or "optionally" means that the subsequently described circumstance may or may not occur, so that the description includes instances where the circumstance occurs and instances where it does not.

"Substantially" or "essentially" means nearly totally or completely, for instance, 90% or greater of some given quantity.

"Transdermal" refers to the delivery of an agent into and/or through the skin for local and/or systemic therapy. The same principles apply to administration through other biological membranes such as those which line the interior of the mouth (e.g. oral mucosa), gastro-intestinal tract, blood-brain barrier, or other body tissues or organs or biological membranes which are exposed or accessible during surgery or during procedures such as laparoscopy or endoscopy.

A material that is "water-soluble" may be defined as soluble or substantially soluble in aqueous solvents. A material that is "water-soluble" preferably dissolves into, within or below the skin or other membrane which is substantially aqueous in nature.

Overview

The present disclosure is directed, at least in part, to the discovery of a biocompatible and water-soluble matrix as described in the claims, wherein the matrix comprises a vaccine active agent and an adjuvant, for use in a microstructure array for transdermally administering the active agent, especially where administration of the vaccine does not cause or substantially does not cause subcutaneous nodules.

Microstructure Arrays

In general, the microstructure array includes a plurality of microstructures as described in the claims. The array includes a backing or planar base where the microstructures extend outwardly from one surface of the backing. At least a portion of the dissolving microstructures provided herein comprises a biocompatible and water-soluble polymer matrix and a vaccine, according to the claims.

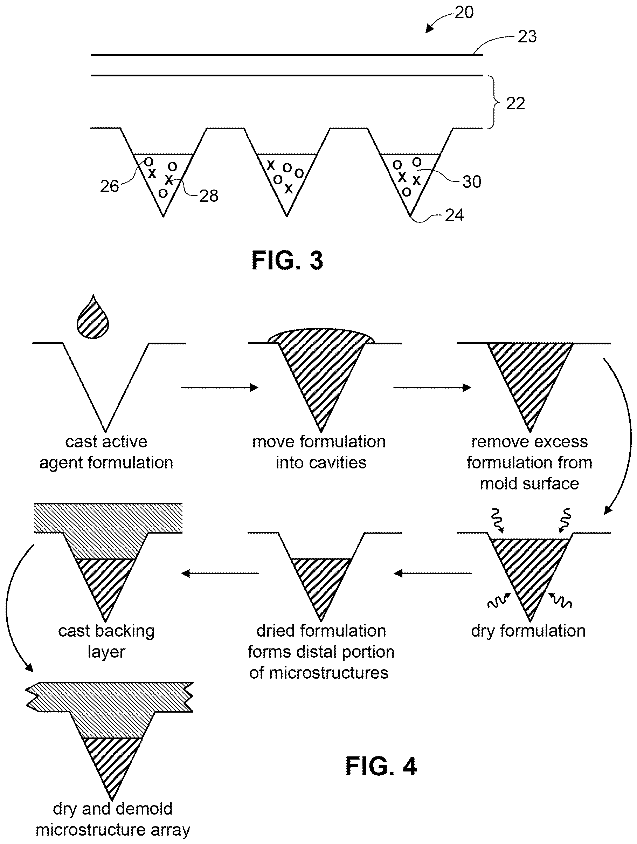

Fig. 3 is an illustration of an exemplary microstructure array 20. The array includes a plurality of microstructures 24 adjacent a backing layer, basement layer or base 22. In other embodiments, the microstructures comprise at least a portion of the backing layer and the distal tip as shown in Fig. 3. The vaccine components are contained at least in a distal portion of the microstructure array. In the microstructures of Fig. 3, each microstructure includes at least one antigen 26 and at least one adjuvant 28 within a biodegradable polymer matrix 30. At least the distal portion and/or tips of the microstructures comprise the biodegradable polymer matrix including the vaccine components. The array may further include a backing substrate 23 adjacent the backing layer. The microstructures may include one or more layers with similar or different compositions. Each of the plurality of microstructures is at least partially formed of a biodegradable polymer matrix. Preferably, at least a distal portion or the distal ends of the microstructures are formed of the biodegradable polymer matrix. The biodegradable polymer matrix comprises at least one structure forming polymer and a vaccine, according to the claims. At least the microstructures distal ends, upon penetration of a subject's skin, undergo dissolution to thereby deliver the vaccine.

The microstructures may comprise one or more additional active agents. In one or more embodiments, at least a portion of the microstructures may include a coating that may optionally contain one or more active agents, that may be the same as or different from the active agent(s) in the microstructures.

In one embodiment, the active agent in the microprojection array is one or more proteins or peptides for use as a vaccine. Vaccines stimulate a body's immune system by inducing pathogen-specific adaptive immunity. Vaccines typically contain at least one antigen and at least one adjuvant, each considered a vaccine active agent herein.

The antigen may be any substance which provokes an immune response. Antigens are usually foreign substances. Non-limiting examples of antigens include proteins, peptides, polysaccharides, or portions thereof. Often, antigens used in vaccines include at least a portion of a bacteria, virus, or other microorganism from which protection is desired. In other embodiments, the antigen may be produced by the bacteria, virus, or other microorganism such as a toxin or protein.

The adjuvant enhances antigen-specific immune responses of the specific antigen. Particulate adjuvants, and insoluble aluminum salts in particular, have been used for more than 80 years. The use of insoluble particulate adjuvants results in formation of nodules at the injection site. A 1955 study found that a nodule formed one day after subcutaneous injection of ovalbumin or diphtheria toxoid when used with aluminum phosphate as an adjuvant (White et al., J Exp Med, 1955, 102(1): 73-82). The presence of the antigen within the nodules for days or weeks gave rise to the "depot theory" that the nodules form an antigen depot at the inoculation site and slowly release the antigen over time providing a priming and a boosting effect (Munks et al., Blood, 2010, 116(24):5191-5199). Thus nodule formation has long been considered a requirement for the action of the adjuvants.

Adjuvant nodules, or granulomas, may be associated with pain, itching, local skin alterations, and systemic symptoms (Avcin et al., Acta Dermatoven APA, 2007, 17(4):182-184). The alterations can include hypertrichosis, eczema, excoriation, and hyperpigmentation (Avcin *et* al.). These nodules can persist for several months or years. Nodule formation varies with the mode of administration. Nodule formation is common when the adjuvant is administered by subcutaneous or intradermal administration as compared to intramuscular injection (Pittman, Vaccine, 2002, S48-S50). Nodule formation is also more common in women as compared to men (Pittman). Thus, administration of vaccines containing aluminum salts has been unfavored for subcutaneous or intradermal administration.

It has recently been discovered that nodule formation is not required for aluminum salts to act as an adjuvant (Munks). While antigen presentation likely depends on small particles of the antigen and the adjuvant, the large nodules that produce discomfort are not necessary (Munks). The present microstructure arrays, therefore, provide insoluble particulate adjuvants that enhance the antigen-specific immune response but do not form nodules with subcutaneous or intradermal administration.

The vaccine active agents may include, for example, those approved in the United States for use against anthrax, diphtheria, hepatitis A, hepatitis B, *Haemophilus influenzae* type a and/or type b, human papillomavirus, influenza, Japanese encephalitis, Lyme disease, measles, meningococcal and pneumococcal diseases, mumps, pertussis, polio, rabies, rotavirus, rubella, shingles, smallpox, tetanus, tuberculosis, typhoid, varicella, and yellow fever. The active agent may comprise live attenuated or killed bacteria, live attenuated viruses, subunit vaccines, conjugate vaccines, synthetic vaccines, viral vectors, polysaccharide vaccines, and DNA vaccines. Among anthrax vaccines, particular preference is given to vaccines comprising the PA (protective antigen), particularly protective antigen which is recombinantly-produced (rPA, i.e., recombinant protective antigen). In one particular, but not limiting, embodiment, at least a portion of the vaccine is a protein based vaccine.

Additional agents include those directed against avian (pandemic) influenza virus, *Campylobacter* sp., *Chlamydia* sp., *Clostridium botulinum, Clostridium difficile,* dengue fever virus, *E. coli,* Ebola virus, Epstein Barr virus, nontypeable *Haemophilus influenzae,* hepatitis C, hepatitis E, herpes viruses including herpes zoster, HIV, leishmanial and malarial parasites, meningococcal serogroup B, nicotine, parainfluenza, ragweed allergen, respiratory syncytial virus (RSV), Rift Valley fever virus, SARS-associated coronavirus, *Shigella* sp., *Staphylococcus aureus,* Streptococcus Group A (GAS), Streptococcus Group B (GBS), tick-borne encephalitis, Venezuelan equine encephalitis, and West Nile virus.

Other agents include those typically used for veterinary uses including vaccines for cats, dogs and other small animals as well as those for use with livestock such as cattle, pigs, goats, horses, chickens, *etc.* Veterinary agents include those directed against parvovirus, distemper virus, adenovirus, parainfluenza virus, *Bortadella bronchiseptica, Leptospira spp., Borrelia burgdorferi,* feline herpesvirus, feline calcivirus, feline panleukopenia virus, feline leukemia virus, feline immunodeficiency virus, and *Chlamydia felis.* Veterinary agents further include those directed against viral respiratory diseases ( infectious bovine rhinotracheitis - IBR, bovine viral diarrhea - BVD parainfluenza-3 virus - PI3, bovine respiratory syncytial virus - BRSV), *Campylobacter fetus* (Vibriosis) *Leptospirosis sp., Trichomoniasis, Moraxella bovis* (pinkeye), Clostridial (Blackleg), Brucellosis, *Mannheimia haemolytica, Pasteurella multocida, Haemophilus somni, Escherichia coli,* Anaplasmosis, foot-and-mouth disease virus (FMDV), procine parvovirus, swine fever, porcine reproductive and respiratory syndrome virus (PRRS), swine influenza, transmissible gastro enteritis virus (TGE), *Staphylococcus hyicus, Actinobacillus pleuropneumonia,* Atrophic rhinitis, Enzootic pneumonia, *Haemophilus parasuis,* Streptococcal meningitis, *Mycoplasma gallisepticum,* Salmonella, Marek's Disease virus, and infectious bronchitis virus. Further veterinary agents include those directed against Eastern/Western Equine Encephalomyelitis, Equine influenza, Potomac Horse Fever, Strangles, Equine Herpesvirus, and Equine Viral Arteritis.

It will be appreciated that the vaccines described herein may include one or more antigens and one or more adjuvants. For example, the seasonal flu vaccine typically includes agents directed against several strains of influenza. Where multiple antigens are included in the vaccine, the antigens may be directed to conferring an immune response to one or more bacteria, virus, or microorganism. For example, more than one antigen specific to the same bacteria, virus or microorganism may be included in the vaccine. Alternatively, multiple antigens specific to different bacteria, viruses, or microorganisms may be included in the vaccine. Where the vaccine includes multiple antigens, the vaccine may include multiple adjuvants. The number of adjuvants included in the vaccine may be the same, less or more than the number of corresponding antigens included in the vaccine. In an embodiment, a vaccine including a single antigen may include multiple adjuvants.

Due to the widespread use of vaccines, vaccine stability is an important consideration when there exists a choice between multiple types of vaccines for a particular condition. For example, in instances in which an active agent is heat sensitive, it is necessary to maintain a temperature-controlled supply chain for the vaccine, often referred to as a "cold chain." Cold chains for vaccines commonly target maintaining the vaccine at 2-8°C. This presents particular difficulties in poor countries with hot climates. Thus, for many vaccines, the solid-state formulation of the microprojection arrays provides enhanced stability and ease of handling over the corresponding liquid vaccines. In other embodiments, the solid-state formulation of the microprojection arrays provides enhanced stability and ease of handling for storage at room temperatures (e.g. 20-25° C).

The microstructure array also includes one or more adjuvants. The adjuvant is one or more insoluble particulate aluminum salt adjuvants. Particular, but not limiting, embodiments include aluminum hydroxide, aluminum phosphate and aluminum potassium sulfate.

The microstructure array may also include additional excipients for inclusion in the biocompatible and water-soluble matrix, including, for example, preservatives, small molecule stabilizers, surfactants, anti-oxidants, and the like.

Microstructure Array Composition

General features of microstructure arrays suitable for use with the formulations and methods provided herein are described in detail in U.S. Patent Publication No. 2008/0269685, U.S. Patent Publication No. 2009/0155330, U.S. Patent Publication No. 2011/0006458, and U.S. Patent Publication No. 2011/0276028. The microstructure array comprises an approximately planar base and attached to the base are a plurality of dissolving microstructures, each having an attachment point to the base and a distal tip to penetrate a subject's skin.

At least a portion of the microstructures comprises a biocompatible and water-soluble matrix comprising one or more hydrophilic, water-soluble polymers. In particular, at least the entire distal portion of the microstructures comprises the biocompatible and water-soluble matrix. The hydrophilic, water soluble polymers include polysaccharides, polyvinyl pyrrolidone, polyvinyl alcohol, polyethylene glycol, copolymers of ethylene glycol and propylene glycol (e.g., Pluronics®), and block copolymers of PLGA and PEG.

The biodegradability of a microstructure array may also be facilitated by inclusion of water-swellable polymers such as crosslinked PVP, sodium starch glycolate, crosslinked polyacrylic acid, crosscarmellose sodium, celluloses, natural and synthetic gums, polysaccharides, or alginates.

The polymer(s) employed may possess a variety and range of molecular weights. The polymers employed are typically polydisperse, such that their molecular weights are actually weight average molecular weights. The polymers may, for example, have molecular weights of at least about 1 kilodalton, at least about 5 kilodaltons, at least about 10 kilodaltons, at least about 20 kilodaltons, at least about 30 kilodaltons, at least about 50 kilodaltons, or at least about 100 kilodaltons, or more. For biodegradable microstructures, it may be desirable to have biodegradable portion(s) comprising one or more polymers having a lower molecular weight, depending upon the selection of polymers. The strength-molecular weight relationship in polymers is an inverse relationship, such that typically, polymers with lower molecular weights exhibit a lower strength and have a tendency to exhibit higher biodegradability and thus are more likely to break due to their lower mechanical strength. In one embodiment, at least the distal layer comprises at least one polymer having a lower molecular weight, e.g., less than about 100 kilodaltons. In another embodiment, at least the distal layer comprises a polymer having a molecular weight less than about 80 kilodaltons.

Exemplary formulations encompass those in which the biocompatible and water-soluble matrix of the dissolving microstructures comprises a polymer as described above having an average molecular weight falling within one of the following ranges: from about 1 -1,000 kDa, from about 5 - 800 kDa, or from about 15 - 700 kDa. For example, for polysaccharides such as dextran, illustrative average molecular weights include 1 kD, 40 kD, 60 kD, and 70 kD. For hydroxyethylstarch or HES, an illustrative average molecular weight is about 600,000 kD, where the molecular weight of the hydroxyethylstarch typically ranges from about 20 kD to about 2,500 kD. One exemplary molecular weight range for hydroxyethylstarch is from about 450 kD to about 800 kD. Illustrative polysaccharides for preparing the biocompatible and water-soluble matrix include dextran 40, dextran 60, dextran 70, tetrastarch and hetastarch.

Exemplary additives and/or excipients may be included in the polymer matrix of the microstructures. Suitable excipients include, but are not limited to, one or more stabilizers, plasticizers, surfactants, chelating agents and/or anti-oxidants.

The microprojection array may include one or more sugars. The biodegradability and/or dissolvability of the microprojection array may be facilitated by the inclusion of one or more sugars. Exemplary sugars include dextrose, fructose, galactose, maltose, maltulose, isomaltulose, mannose, lactose, lactulose, sucrose, and trehalose. The sugar may also be a sugar alcohol, for example, lactitol, maltitol, sorbitol, mannitol, glycerol, xylitol, galactitol, and erythritol. Cyclodextrins can also be used advantageously in microneedle arrays, for example $\alpha$, $\beta$, and $\gamma$ cyclodextrins, for example hydroxypropyl-$\beta$-cyclodextrin and methyl-$\beta$-cyclodextrin. Particularly preferred are sugar alcohols, preferably acyclic polyhydric linear sugar alcohols, which, when combined with a polysaccharide as described above, appear to be particularly effective in both stabilizing the active agent components (e.g., peptides and proteins or protein fragments) in the dried state, and for enhancing the mechanical properties of the microprojections by exhibiting a plasticizing-like effect on the polysaccharide polymer component. One particularly preferred sugar alcohol in this regard is sorbitol.

One or more surfactants may be added to the casting solution to change the solutions' surface tension and/or reduce the hydrophobic interactions of proteins. Any suitable surfactant as known in the art may be used. Exemplary surfactants include, but are not limited to, emulsifiers such as Polysorbate 20 and Polysorbate 80. In another embodiment, the choice of solvent, or addition of a solvent, in the formulation solution or suspension is used to improve the spreading and/or loading of the formulation in a mold.

The casting solution or formulation includes one or more co-solvents, as claimed, to improve spreading and movement of the formulation over the mold and/or over and into the mold cavities. Furthermore, the co-solvents may also or alternatively improve the solubility of either antigen or adjuvant. In one embodiment, about 1-25% of a co-solvent is included in the formulation. In embodiments, about 1-20%, 1-15%, 1-10%, 1-5%, 5-20%, 5-15%, 5-10%, 10-20%, 10-15%, or 15-20% of a co-solvent is included in the formulation. One exemplary co-solvent is isopropyl alcohol. Further exemplary co-solvents include ethyl alcohol, methanol, and butanol. In one embodiment, the co-solvent improves spreading and/or movement of the formulation by decreasing the contact angle of the formulation on the mold surface. In embodiments, the contact angle is less than about 100°. In other embodiments, the contact angle is less than about 90°. In other embodiments, the contact angle is less than about 90-100°. As described in Example 1, inclusion of 10 wt% isopropyl alcohol decreased the contact angle of the formulation on the mold from 110 degrees to 79 degrees, a reduction of nearly a third of the original angle.

One or more antioxidants may be added to the casting solution. Any suitable antioxidant as known in the art may be used. Exemplary antioxidants include, but are not limited to, methionine, cysteine, D-alpha tocopherol acetate, EDTA, and vitamin E.

The microstructure formulations provided herein are meant to encompass the formulations both in dried form, e.g., in the microstructures themselves, and in liquid form, e.g., for preparing the microstructures. More specifically, the liquid formulations include components as described above in an aqueous buffer. Exemplary buffers include phosphate buffered saline and histidine.

The distal layer (i.e., microstructure or microneedle layer) may comprise one or more polymers having a lower molecular weight while the proximal layer and/or the backing or base may comprise polymers having a higher molecular weight. The polymers for the distal and/or proximal portions may be selected based at least partly on the molecular weight of the polymers to facilitate separation or detachment of at least a portion of the microstructures upon administration.

Generally, the number of microstructures forming the plurality in the array is at least about 50, preferably at least about 100, at least about 500, at least about 1000, at least about 1400, at least about 1600, at least about 2000, at least about 3000, or at least about 4000. For example, the number of microstructures in the array may range from about 1000 to about 4000, or from about 1000 to about 3000, or from about 2000 to about 4000, or from about 2000 to about 3500, or from about 2000 to about 3000, or from about 2200 to about 3200. The area density of microstructures, given their small size, may not be particularly high, but for example the number of microstructures per $cm^2$ may be at least about 50, at least about 250, at least about 500, at least about 750, at least about 1000, at least about 2000, or at least about 3000 microstructures per $cm^2$ or more.

While the array itself may possess any of a number of shapes, the array is generally sized to possess a diameter of from about 5 millimeters to about 25 millimeters, or from about 7 to about 20 millimeters, or from about 8 to about 16 millimeters. Exemplary diameters include 5, 6, 7, 8, 9, 10, 11, 12, 13, 14, 15, 16, 17, 18, 19, 20, 21, 22, 23, 24, and 25 millimeters.

The sizes of the microneedles and other protrusions are a function of the manufacturing technology and of the precise application. In general, however, microstructures and other microprotrusions used in practice may be expected to have a height of at least about 20 to about 1000 microns, more preferably from about 50 to about 750 microns and most preferably from about 100 to about 500 microns. In specific but not limiting embodiments, the microstructures have a height of at least about 100 $\mu$m, at least about 150 $\mu$m, at least about 200 $\mu$m, at least about 250 $\mu$m, or at least about 300 $\mu$m. In general it is also preferred that the microprojections have a height of no more than about 1 mm, no more than about 500 $\mu$m, no more than about 300 $\mu$m, or in some cases no more than about 200 $\mu$m or 150 $\mu$m. Generally, the microprotrusions are long enough to penetrate at least partially through the stratum corneum layer of skin at some suitable point of application to a subject, e.g., a mammalian subject, for example the thigh, hip, arm, or torso. The microprojections may have an aspect ratio of at least 3:1 (height to diameter at base), at least about 2:1, or at least about 1:1.

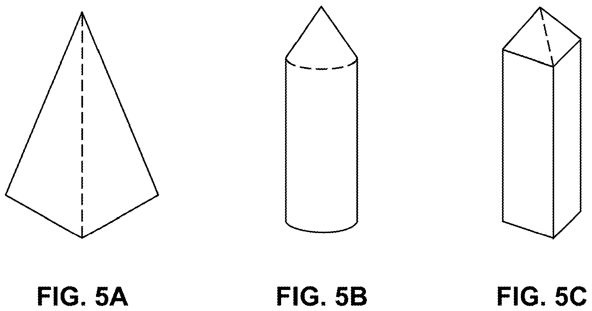

The microprojections may possess any suitable shape including, but not limited to polygonal or cylindrical. Particular embodiments include pyramidal including a four-sided pyramid, a funnel shape, a cylinder, a combination of funnel and cylinder shape having a funnel tip and a cylindrical base, and a cone with a polygonal bottom, for example hexagonal or rhombus-shaped. One illustrative shape for the microstructures is a cone with a polygonal bottom, for example, being hexagonal or rhombus-shaped. Additional possible microprojection shapes are shown, for example, in U.S. Published Patent App. 2004/0087992. In embodiments, at least a portion of the microstructure shape may be substantially cylindrical, cone-shaped, funnel-shaped, or pyramidal. Microprojections may in some cases have a shape which becomes thicker towards the base, for example microprojections which have roughly the appearance of a funnel, or more generally where the diameter of the microprojection grows faster than linearly with distance to the microprojection distal end. It will be appreciated that polygonal microprojections may also have a shape which becomes thicker toward the base or where a radius or diameter grows faster than linearly with distance to the microprojection distal end. Where microprojections are thicker towards the base, a portion of the microprojection adjacent to the base, which may be called the "foundation," may be designed not to penetrate the skin. In further embodiments, at least a portion of the microstructures has an asymmetrical cross-dimensional shape. Suitable asymmetric shapes include, but are not limited to, rectangular, square, oval, elliptical, circular, rhombus, triangular, polygonal, star-shaped, etc. In some embodiments, the distal layer has a cross-dimension in one direction that is smaller than the cross-dimension in the other direction. Exemplary cross-dimensional shapes with this configuration include, but are not limited to, rectangular, rhombus shaped, ellipse, and oval (see FIGS. 5A-5E for non-limiting examples). It will further be appreciated that different portions and/or layers of a microstructure may have different cross-dimensional shapes. At least a portion of the microstructures may include one or more blade or piercing elements along its length and/or at the distal tip. In some preferred embodiments, at least a portion of the microstructures have a sharp, pointed, or spike-shaped distal end.

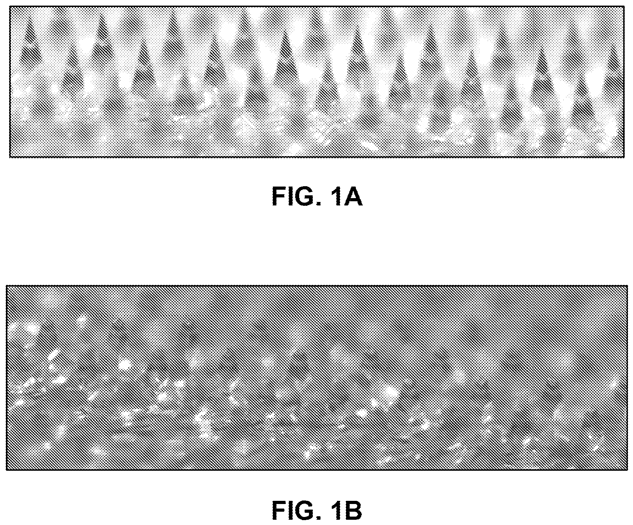

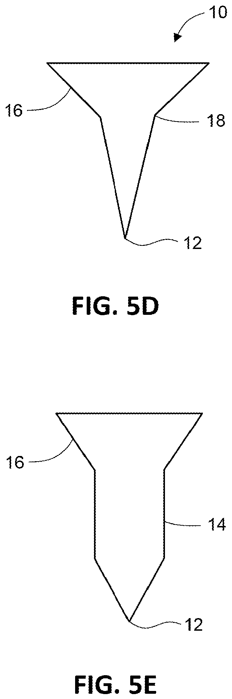

In some embodiments, microstructure shape can be understood in terms of a tip, a shank and a funnel. The angle at the tip is the apex angle - included angle by the planes or cone - and can have values from about 5 degree to about 60 degrees. The straight or substantially straight shank may or may not be present in a particular microstructure design. At the base of the shank or tip, towards the distal end, the included angle has a discontinuity or a point of inflection. The included angle jumps to take on a value greater than the apex angle for a shank-less tip and to greater than 0 degrees for microstructures with a shank. Portions of the microstructure beyond this point of inflection may be referred to as a "funnel". FIGS. 5D and 5E show examples of cross sectional elevation of the microstructures 10 delineating different regions including the tip 12, shank 14, inflection point or edge 18 and the funnel 16. The funnel allows for a greater fill amount to be used in forming the arrays and a resulting greater volume of or portion of the arrays including the active agent. In Fig. 5D, the diameter of the microstructure is growing faster than linear fashion with respect to the distance from the distal end. Fig. 1A shows a microstructure array comprising a plurality of microstructures formed in accord with Example 2. The microstructures of Fig. 1A have a sharp distal end. As in Fig. 5D, the diameter of the microstructures of Fig. 1A grow faster than linearly moving from the distal tip to the proximal end. Where microstructures are thicker towards the base, a portion of the microstructure adjacent to the base, which may be referred to herein as a "proximal portion", "backing portion", "basement", "foundation", or as an "upper portion", may be designed not to penetrate the skin. In this manner, the basement portion or region may be used to limit the penetration depth of the microstructures.

The proximal funnel shape allows for relatively larger volumes to be dispensed in the microstructure mold for a given total length of the microstructure. The proximal funnel shape provides a larger volume (to fill) without requiring a proportional increase in microstructure height or height of the portion of the microstructures containing the active agent, which results in a longer drug containing portion in the microstructure. Thus, the proximal funnel shape allows for a larger solid volume for the distal portion of the microstructure with a single fill of the mold. Other shapes may require several fill and dry cycles to achieve the same amount of solid distal portion as one fill and dry cycle for the funnel shaped microstructures.

In one exemplary embodiment, at least a portion of the microstructures have a cylindrical funnel shape as seen, for example, in Fig. 5E. Microstructures with this shape have a cylindrical shank 14 and an optional funnel 16 at the proximal end. In this embodiment, the distal tips of the microstructures typically, but not always, have a sharp, pointed or conical distal end 12 to ease and/or facilitate penetration. The microstructures may further have a funnel shape at the proximal end and a cylindrical shank between the distal and proximal ends. It will be appreciated that the funnel need not have a "funnel" shape. Instead, the funnel section may have any shape that allows for greater fill of a mold and/or modification of penetration of the microstructures. For example, the funnel section may have a polygon shape where the diameter of the polygon grows faster than linearly moving from the inflection point to the proximal end.

The funnel portion may also be used to limit the depth of penetration. Since the funnel has a several times higher volume per unit height than the tip or shank, it also requires several times higher energy to penetrate per unit depth than the tip or shank. Hence for a given energy, the microstructure may penetrate no more than the length of the tip and shank. The funnel thus can effectively act as the design element in the microstructure that limits the depth of penetration thereby ensuring tolerable sensation. It will be appreciated that other proximal end shapes may be used to limit or otherwise affect penetration of the microstructures. This is true especially where the proximal end has a larger diameter or cross-section than the shaft or middle section of the microstructures.

In one or more embodiments, the microstructures have a sharp point or tip. A tip diameter of less than about 5 $\mu$m or 2 $\mu$m may be desirable. A tip diameter of less than about 1.5 $\mu$m is preferred, as is a tip diameter of less than about 1 $\mu$m.

The microprojections are typically spaced about 0-500 μm apart. In specific, but not limiting embodiments, the microprojections are spaced about 0 μm, about 50 μm, about 100 μm, about 150 μm, about 200 μm, about 250 μm, about 300 μm, about 350 μm, about 400 μm, about 450 μm, or about 500 μm apart. The space between the microprojections may be measured from the base of the microprojections (base to base) or from the tip (tip to tip).

In further embodiments, at least a portion of the microprojections are detachable from the microprojection array. Detachable microprojection arrays are described in U.S. Patent Publication 2009/0155330 and in U.S. Patent Application No. 61/745,513. Detachable microprojection arrays may be accomplished by a number of approaches including, but not limited to, a layered approach in which the array is composed of multiple layers, and a layer comprising the areas where the microprojections attach to the base of the array is more readily degradable than other layers.

One advantage of detaching microprojections is the elimination of sharp disposal requirements, while another is elimination of needle stick injury. Additionally, detaching microprojections may advantageously substantially reduce or eliminate misuse, for example, needle sharing, since the substrate or base absent the microprojections or with microprojections whose tips have been blunted due to biodegradability will not penetrate the skin. Another advantage of detaching microprojections is the avoidance of drug misuse, since the drugenriched tips are dissolved in the skin, leaving no or minimal drug remaining in the array postadministration.

Alternatively, an array made of a homogeneous material may be employed, in which the material is more readily degradable at lower pH's. Arrays made of such a material will tend to degrade more readily near the attachment points because these, being closer to the surface of the skin, are at a lower pH than the distal ends of the microprojections. (The pH of the skin's surface is generally lower than that of the skin further inwards, pH being for example approximately 4.5 on the surface and approximately 6.5 to 7.5 inward).

Materials whose solubility is dependent on pH can be, for example, insoluble in pure water but dissolve in an acidic or basic pH environment. Using such materials or combination of materials, the arrays can be made to differentially biodegrade at the skin surface (pH approximately 4.5) or inside the skin. In the former embodiment, the whole array can biodegrade, while in the latter, the microprojection portion of the array will biodegrade allowing the base substrate to be removed and discarded. In a preferred embodiment, the microstructure array corresponds to the latter, wherein the microprojection portion of the array dissolves and biodegrades upon administration of active agent, allowing the base substrate to be removed and discarded.

Materials whose degradability in an aqueous medium is dependent on pH may be made, for example, by utilizing the acrylate copolymers sold by Rohm Pharma under the brand name Eudragit®, which are widely used in pharmaceutical formulations. A further example of a material with pH-dependent solubility is hydroxypropyl cellulose phthalate. Materials with pH-dependent solubility have been developed, for example, for use as enteric coatings in oral dosage forms. See, e.g., U.S. Patent No. 5,900,252 and Remington's Pharmaceutical Sciences (18th ed. 1990).

It may also be desirable, in certain instances, for the microprojection arrays provided herein to comprise one or more additional layers in addition to the biocompatible and water-soluble matrix layer which comprises a polymer such as a polysaccharide, a sugar alcohol, and the active agent. There are a number of reasons why arrays with multiple layers may be desirable. For example, it is often desirable that, compared to the whole volume of the microprojection array, the microprojections themselves possess a higher concentration of active ingredient such as an active agent. This is so, for example, because the microprojections can be expected, in many cases, to dissolve more rapidly, being in a more hydrated environment than the base of the array. Furthermore, in certain protocols for array application, the array may be left in for a short period of time during which essentially only the microprojections can dissolve to a substantial extent. The desirability of placing a higher concentration of active agent in the projections themselves is particularly acute when the active is costly. One way to achieve a higher concentration of active in the projections themselves is to have a first active-containing layer which includes the microprojections or a substantial proportion of the microprojections, and a second layer with a reduced or zero concentration of active which includes the base or a substantial proportion of the base.

Generally, in a preferred microstructure array configuration comprising two or more different layers, i.e., a layer comprising a plurality of microstructures or projections, and a base or backing layer supporting the microstructures, the base layer comprises a biocompatible, non-water soluble rand non-biodegradable matrix. Once the microstructure array penetrates the skin, the microstructure (tip portions) dissolve, thereby delivering the active agent transdermally. The base layer comprises any of a number of biocompatible, non-water soluble polymers including polyesters, polyaminoacids, polyanhydrides, polyorthoesters, polyurethanes, polycarbonates, polyetheresters, polycaprolactones (PCL), polyesteramides, and copolymers thereof. Illustrative polymers include polyacrylates, celluloses, poly(lactic acid) (PLA), poly(glycolic acid) (PGA), poly(lactic acid-co-glycolic acid)s (PLGAs), poly(butyric acid), poly(valeric acid). An exemplary backing or base layer comprises poly-lactide-poly-glycolide (PLGA 75/25 or PLGA 50/50). In some embodiments, an exemplary backing or base layer comprises PLGA having at least about 50% lactide content. See, e.g., Example 4 which describes casting a liquid backing layer formulation comprising PLGA (75/25) over formulation comprising a vaccine active agent dried in the mold. In embodiments, the polymer for use in the backing or base layer has a degradation half-life of at least 1-24 hours when placed in or on skin or other biological membrane.

In another embodiment, the backing or base layer comprises an adhesive or other layer that is pre-formed and applied to the microstructures. In further embodiments, the backing layer is formed from a liquid adhesive that is cast onto the dried formulation comprising a vaccine active agent dried in the mold. These adhesives are typically cured rather than requiring removal of solvent. Suitable adhesives include, but are not limited to the Dymax® UV-curable 1128A-M, 1161-M, 1162-M, 1165-M, 1180-M, and 1187-M medical device adhesives available from Dymax. It will be appreciated that any biocompatible adhesive is suitable for use with, in and/or as the backing layer. The backing layer may also be a nonwoven or porous film double coated with pressure sensitive adhesive.

The microstructure arrays should have sufficient mechanical strength to at least partially penetrate the stratum corneum or other membrane surface of a subject. It will be appreciated that different mechanical strength will be required for application at different sites. One method for assessing mechanical strength is a skin-penetration efficiency (SPE) study as described in Example 7. Preferably, the arrays have a SPE of about 50-100%. In other embodiments, the arrays have a SPE of about 50-80%, about 50-85%, about 50-90%, about 50-95%, about 60-80%, about 60-85%, about 60-90%, about 60-95%, about 60-100%, about 75-80%, about 75-85%, about 75-90%, about 75-95%, about 75-100%, about 80-85%, about 80-90%, about 80-95%, about 80-100%, about 90-95%, and about 90-100%. In specific, non-limiting, embodiments, the arrays have a SPE of about 50%, 60%, 70%, 75%, 80%, 85%, 90%, 95%, 99%, and 100%.

Preferably, at least about 50-100% of the active agent is delivered by the MSAs described herein. Delivery efficiency may be determined by preparing the MSA and applying the MSA *in vivo* or *in vitro*. An in vitro method of determining delivery efficiency includes immersing MSA in an aqueous extraction medium for a period of time, e.g. 30 minutes) as described in Example 7. The extraction medium is then analyzed for the agent. One analysis method is SEC-HPLC. The apparent delivered dose per unit and delivery efficiency are calculated with the formulas:

$$\text{Apparent delivered dose} = \text{initial drug load} - \text{residual drug}$$

$$\%\text{Drug delivery efficiency} = 100 \times \text{Apparent delivered dose/initial drug load.}$$

In embodiments, the MSA has a delivery efficiency of at least about 50-60%, about 50-70%, about 50-75%, about 50-80%, about 50-90%, about 50-95%, about 50-99%, about 60-70%, about 60-75%, about 60-80%, about 60-90%, about 60-95%, about 60-99%, about 70-75%, about 70-80%, about 70-90%, about 70-95%, about 70-99%, about 75-80%, about 75-90%, about 75-95%, about 75-99%, about 80-90%, about 80-95%, about 80-99%, about 90-95%, about 90-99%, or about 95-99%. In specific, but not limiting, embodiments the MSA has a delivery efficiency of at least about 50%, 60%, 70%, 75%, 80%, 90%, 95%, 99%, or 100%.

Active Agents

The microstructure array of the invention comprises a vaccine active agent. It is generally, but not always, desirable that the concentration of active agent by weight in the microprojection arrays is comparatively high, since it allows a higher concentration of active agent to be presented to the individual upon insertion of the microprojections into the skin. Illustrative concentrations in the solids forming the array (the biocompatible and water-soluble matrix) are as follows: at least about 0.1 %, 0.5%, 1%, 2%, 5%, 10%, 15% or 20% by weight active agent, i.e. a vaccine. More preferably, the weight percent solids in the biocompatible and water-soluble matrix forming the microstructure projections range from about 1-15% active agent. That is to say, exemplary percentages by weight active agent, i.e. a vaccine, in the plurality of solid microprojections include 1%, 2%, 3%, 4%, 5%, 6%, 7%, 8%, 9%, 10%, 11%, 12%, 13%, 14%, and 15% or greater. For the corresponding liquid formulations, the amount of active agent will generally range from about 0.05 wt% to about 10 wt% active agent, or preferably, from about 0.1 wt% to about 5 wt% active agent. In specific, but not limiting embodiments, the amount of active agent in the liquid formulations is about 0.1 wt%, about 0.2 wt%, 0.25 wt%, 0.3 wt%, 0.4 wt%, 0.5 wt%, 0.6 wt%, 0.7 wt%, 0.75 wt%, 0.8 wt%, 1 wt%, 2 wt%, 3 wt%, 4 wt%, 5 wt%, 6 wt%, 7 wt%, 8 wt%, 9 wt%, or 10 wt%. per $cm^2$.

The dose that is delivered to the body is that appropriate to elicit a substantial therapeutic and/or immune response in a large majority of individuals. In general, a desirable dose is at least about 0.1 pg/$cm^2$, at least about 0.5 pg/$cm^2$, at least about 1 pg/$cm^2$, at least about 2 pg/$cm^2$, at least about 5 pg/$cm^2$, or at least about 10 $\mu$g/$cm^2$.

Alternatively, the active agent dose may be measured as a percentage of the dose delivered by other paths, for example intramuscularly. It may be desirable, for example, to deliver at least about 1 %, at least about 10%, at least about 25%, at least about 50%, at least about 75%, at least about 100%, at least about 150%, or at least about 200% of the dose delivered by other paths, for example of the dose delivered intramuscularly or intradermally via a syringe. Alternatively, it may be desired to deliver no more than about 200%, no more than about 150%, no more than about 100%, no more than about 75%, no more than about 50%, no more than about 25%, no more than about 10%, or no more than about 1% of the dose delivered by other paths. As with conventional transdermal patches, dose delivery (DDE) by a microprojection array may be less than the total active agent content of the microprojection arrays.

Manufacturing the Microproiection Arrays

Microprojection arrays can be fabricated by employing the techniques for the fabrication of two-layer arrays described in U.S. Patent Publication No. 2008/0269685.

Examples of forming various microstructure arrays using exemplary formulations are provided in Examples 1-5. The microstructure array of the invention is prepared according to the method described in the claims. The terms casting solution, formulation, and polymer solution or suspension are used interchangeably herein and discussion or reference to one is intended to include and apply to each and all terms. The formulation may also include excipients including, but not limited to, surfactants, sugars, degradation enhancers, chelating agents, and anti-oxidants. The formulation includes one or more co-solvents. Where the active agent and polymers are separately mixed, the excipients may be mixed with the active agent and/or the polymer. It will be appreciated that some of the excipients may be mixed with the active agent while others are mixed with the polymer. Further, the excipients may be separately mixed and added to the active agent solution, the polymer solution, or the mixed active agent/polymer solution or suspension. In embodiments, the mold is purged with a soluble gas prior to casting the polymer solution or suspension on the mold. The method further includes removing excess solution, suspension or formulation on the mold surface. Typically, excess formulation is scraped or wiped from the mold surface. Excess formulation is removed from the mold surface prior to drying or otherwise removing solvent. The solvent or solvent mixture may be removed by any suitable means including, but not limited, to drying the mold filled with the casting solution, formulation or suspension. In an embodiment, the mold filled with the casting solution, formulation or suspension is placed in a suitable oven for drying. In an embodiment, drying or removing solvent comprises placing the mold in an oven at about 5°C to 50°C. The microprojections themselves comprise the active agent, as opposed to having the active agent present as a coating on a microprojection or microneedle made of a biocompatible material such as a metal. Where the microstructures are not integral with a substrate and/or backing layer, the microstructures are typically affixed to the substrate and/or backing layer with an adhesive prior to de-molding.

The molds used to form the arrays in the methods herein can be made using a variety of methods and materials. Exemplary molds and methods of making molds are described, for example, in U.S. Patent Publication No. 2008/0269685. In one exemplary embodiment, the mold is a negative mold formed from a silicone such as polydimethylsilicone. A negative mold is typically formed by preparing a master microprojection array and casting a liquid mold material over the master array. A microstructure array tool having different geometries can be used to make the negative mold (generally but not necessarily using polydimethylsilicone). Additional negative mold materials include polyurethanes, ceramic materials, waxes, and the like. This mold is then used to fabricate a microstructure array (MSA) which replicates the geometry of the original tool. One exemplary tool possesses a diamond shape with a microprojection height of about 200 μm, a base width of about 70 μm, and a projection-to-projection spacing of about 200 μm as described in Example 6. The mold is allowed to dry and harden, which results in a mold comprising cavities corresponding to the microprojections of the master array. It will be appreciated that the molds suitable for use in the present methods may be prepared according to other methods.

Turning back to the method of preparing a microstructure array, an array of microprotrusions or microprojections is generally formed by (a) providing a mold with cavities corresponding to the negative of the microprotrusions, (b) casting a solution comprising components suitable for forming a biocompatible and water-soluble matrix, the active agent, and a solvent onto the mold, (c) removing the solvent, and (d) demolding the resulting array from the mold. The method of the invention is, more specifically, according to the claims. Example 1 provides exemplary liquid formulations for the casting formulations. Although the formulations shown in Example 1 do not include antigen as the vaccine active agent, it will be appreciated that one or more antigens will typically be included in the liquid casting formulations. These liquid formulations comprise a combination of dextran, sorbitol, and an aluminum salt ("alum") as an adjuvant. Formulations 2 and 4 additionally include isopropyl alcohol as a surfactant to lower the surface tension between the formulation and the mold surface. Filling of the mold may be affected by the surface tension and/or viscosity of the formulation. For example, aluminum hydroxide has a polar surface, which produces a higher surface tension when used with a non-polar silicone mold. In embodiments shown in Example 1, the formulations include about 10 wt% of isopropyl alcohol. The surfactant reduces the surface tension of the formulation allowing better flow of the formulation over the mold surface, which allows the formulation to flow into the cavities more effectively. Reducing the contact angle of the formulation on the mold surface decreases the flow resistance of the formulation on the mold. Addition of a surfactant can reduce the contact angle of the formulation and thereby improve flow. As seen in Example 1, inclusion of 10-15 wt% of isopropyl alcohol as a surfactant reduced the contact angle of the formulation from 110° to 79°, a reduction of 31° (about 28% reduction in the contact angle). As seen in Example 1, formulations that contain 10% of a surfactant such as isopropyl alcohol (IPA) (formulation 2 in Table 1) spread much better as compared to formulations without IPA (formulation 1) as evidenced by the reduction in contact angle between the formulation and the mold surface. Other surfactants would be expected likewise to reduce the contact angle of the formulation on the mold surface. Only aqueous liquid casting formulations comprising a vaccine active agent, an insoluble particulate aluminum salt adjuvant, a hydrophilic polymer and a co-solvent in an aqueous buffer, as claimed, are according to the invention.

A liquid active agent formulation as described above is formed by generally mixing a vaccine active agent, an adjuvant as claimed, a polymer as claimed, a co-solvent as claimed and optionally other excipients or additives, in an aqueous buffer.

The formulation is dispensed onto the mold surface and/or into the mold cavities. The mold is then filled using any of a number of suitable techniques, such as wiping, compression, pressurization, and the formulation is moved into the cavities by any suitable means. In one embodiment, the formulation is dispensed on the mold surface. The mold surface is wiped with an edged tool and the formulation is moved into the cavities as the formulation is wiped across the mold. In another embodiment, the mold surface with solution thereon is covered to spread the solution or formulation on the mold and at least partially into the cavities. In yet other embodiments, the solution is spread on the mold without covering. Excess solution may be wiped or otherwise removed from the mold surface. In another embodiment, a soluble gas is used to move the casting solution into or further into the cavities. Specific, but not limiting, soluble gases are $CO_2$ and $CH_4$.

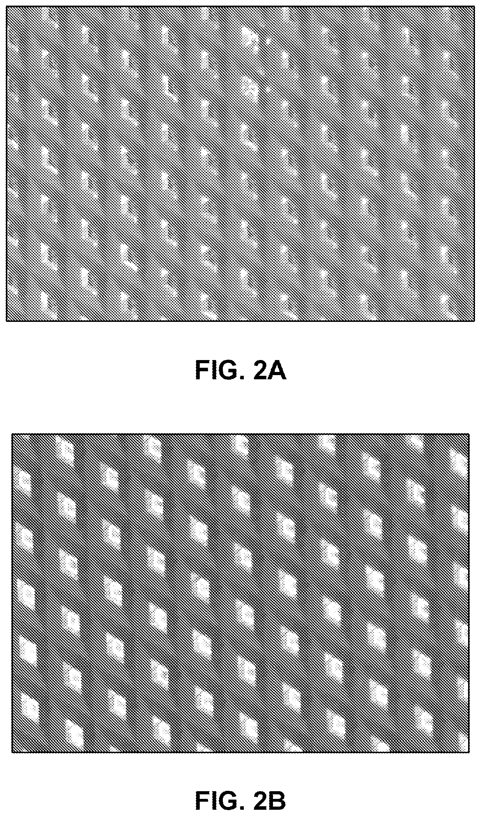

In a further embodiment, the mold is pressurized, with or without a cover, to move the solution into or further into the cavities of the mold. Pressurization may be used where the formulation is dispensed onto the mold surface and/or where the formulation is dispensed into the cavities. Pressurization may be accomplished by placing the mold with the casting solution into a pressure vessel as known in the art. Pressurization may involve a pressure of at least about 3 psi, about 5 psi, about 10 psi, about 14.7 psi, about 20 psi, about 30 psi, about 40 psi, or about 50 psi above atmospheric. In other embodiments, pressurization involves a pressure of at least about 3-50 psi above atmospheric. In other embodiments, pressurization involves a pressure of at least about 3-40 psi, about 3-30 psi, about 3-20 psi, about 3-14.7 psi, about 3-10 psi, about 3-5 psi, about 5-50 psi, about 5-30 psi, about 5-20 psi, about 5-14.7 psi, about 5-10 psi, about 10-50 psi, about 10-30 psi, about 10-20 psi, about 10-14.7 psi, about 20-50 psi, about 20-30 psi, or about 30-40 psi above atmospheric, wherein 1 psi corresponds to 6.89 kPa. As described in Example 2 and shown in Figs. 1A-1 B and 2A-2B, pressurizing the mold prior to drying pushes or draws the liquid formulation into the cavities before the drying process begins. As seen in Fig. 2A, the formulation fill level is lower after pressurization than the formulation fill level in cavities without pressurization (see Fig. 2B). The lower fill level of the cavities indicates that the formulation has moved further into the cavity, especially the cavity tip. This is further evidenced by Fig. 1A, which shows microstructures that were formed using a pressurization step prior to drying. As seen in the figure, the microstructures have sharp, defined edges that indicate the formulation fully contacted the mold surface, even at the distal tips. In contrast, as seen in Fig. 1B, the microstructures formed without a pressurization step are trunked and irregular, which indicates the mold cavities were not filled with formulation leaving air gaps.

Pressure may be applied for a period of time suitable to push or draw the formulation into the mold cavities. In embodiments, pressure is applied for at least about 5 seconds to about 5 minutes. In other embodiments, pressure is applied for at least about 5 seconds to 4 minutes, about 5 seconds to 3 minutes, about 5 seconds to 2 minutes, about 5-90 seconds, about 5-60 seconds, about 5-30 seconds, about 5-15 seconds, about 30 seconds to about 5 minutes, about 30 seconds to 4 minutes, about 30 seconds to 3 minutes, about 30 seconds to 2 minutes, about 30-90 seconds, about 30-60 seconds, about 1-5 minutes, about 1-4 minutes, about 1-3 minutes, about 1-2 minutes, about 60-90 seconds, about 90 seconds to about 5 minutes, about 90 seconds to 4 minutes, about 90 seconds to 3 minutes, about 90 seconds to 2 minutes, about 2-5 minutes, about 2-4 minutes, about 2-3 minutes, about 3-5 minutes, about 3-4 minutes, or about 4-5 minutes. In specific, but non-limiting embodiments, pressure is applied for at least about 5 seconds, 10 seconds, 15 seconds, 20 seconds, 30 seconds, 45 seconds, 60 seconds, 90 seconds, 2 minutes, 3 minutes, 4 minutes, or 5 minutes.

The mold may be treated prior to dispensing the casting solution to improve dispensing of the casting solution and/or to avoid or reduce the presence of air bubbles. In embodiments, the mold, or portions thereof, is treated to improve the ability of the casting solution to wet the mold. Suitable treatments are known in the art and described, for example, in U.S. Patent Publication No. 2008/0269685. In addition, or separately, the casting solution may include ingredients to prevent, reduce, or minimize bubbling. One exemplary ingredient is an anti-foaming agent. Another embodiment of a surface treatment includes coating at a least a portion of the mold with a substance that improves the ability of the casting solution or suspension to wet the mold surface. In non-limiting embodiments, at least a portion of the mold surface is coated with at least one of calcium carbonate, ethyl acetate, a silicone fluid, or oxygen plasma.

After moving the formulation into the cavities, the liquid formulation contained in the mold is dried in either one or multiple primary drying steps, depending, for example, on the physicochemical properties of the respective liquid formulations, such as viscosity, solids content, surface interaction between liquid formulation and mold, etc. In one step primary drying, the liquid formulation contained in the mold is directly placed in an incubator oven at a temperature ranging from about 25°C to about 40°C to remove water. The one step drying can take place anywhere from 20 minutes to several hours. In a two-step drying process, the first step is a slow drying step in which the liquid formulation-filled mold is dried under controlled humidity and/or under pressure. In one embodiment, the mold is first placed in a controlled humidity chamber, e.g. with a relative humidity of about 10-95% or 75-90% RH at a temperature of about 5-50° C, for about 1 min to 60 minutes. In another embodiment, the mold is initially dried in a chamber having a partial pressure of water of about 1.33 mPa (0.01 mTorr) to about 30.66 kPa (230 Torr) at a temperature of about 5-50° C or about 10-50° C. The initial drying step is followed by placement of the mold in an incubator oven at a temperature ranging from about 5-50° C for about 20 minutes to several hours.

In another embodiment, the mold with the formulation is dried from beneath, under or below the mold. It will be appreciated that the formulation may be dried from substantially beneath, under or below the mold. The under method of drying has a benefit of reducing time necessary for drying. In embodiments, the microstructure formulation is dried from underneath for 5-30 minutes. In other embodiments, the formulation is dried from underneath for 5-25 minutes, 5-20 minutes, 5-15 minutes, 5-10 minutes, 10-25 minutes, 10-20 minutes, 10-15 minutes, 15-25 minutes, 15-20 minutes, or 20-25 minutes. In specific embodiments, the formulation is dried from underneath for about 5, 10, 15, 20, 25, or 30 minutes. In embodiments, the mold is heated to maintain or substantially maintain the temperature of the formulation at about 5-50°C. The formulation may be dried from below using conductive and/or radiative heating. In embodiments, the mold surface is heated from below. It will be appreciated that the parameters including, but not limited to, temperature, time, and equipment as described above are contemplated and intended to apply to the under drying method.

The secondary drying step(s) may be performed under vacuum. Drying methods are further described in U.S. Publication No. (Attorney Docket No. 091500-0501/8131.US00).

Following drying, a backing layer may be cast on the dried formulation-containing mold to thereby attach to the plurality of microprojections. In another embodiment, the backing layer is otherwise affixed to the plurality of microprojections.

In one embodiment, a liquid backing formulation is dispensed onto the mold or into the cavities. The liquid backing formulation is typically prepared by dissolving or suspending one or more polymers in a suitable solvent. In a preferred embodiment, the one or more polymers are biocompatible. Typically, but not always, the polymers are non-biodegradable. In another embodiment, the backing formulation may comprise one or more biodegradable and/or non-biodegradable polymers. Suitable biodegradable polymers are described above. Suitable non-biodegradable polymers are known in the art and include, but are not limited to, amphiphilic polyurethanes, polyether polyurethane (PEU), polyetheretherketone (PEEK), poly(lactic-co-glycolic acid) (PLGA), polylactic acid (PLA), polyethylene terephthalate, polycarbonate, acrylic polymers such as those sold under the trade name Eudragit®, polyvinylpyrrolidones (PVP), polyamide-imide (PAI), and/or co-polymers thereof. Further suitable polymers are described in U.S. Patent No. 7,785,301. In an embodiment, the components of the microprojections (i.e., the components of the formulation) are not soluble in the solvent used in the backing layer. Generally, the solvent used in casting the backing layer is an organic solvent such as acetonitrile, ethanol, isopropyl alcohol, ethyl acetate, and the like. An exemplary backing formulation is described in Example 3.

In further embodiments, the backing layer is a non-solvent based liquid adhesive. These adhesives will be cured rather than requiring removal of solvent. Suitable adhesives include, but are not limited to the Dymax® UV-curable 1128A-M, 1161-M, 1162-M, 1165-M, 1180-M, and 1187-M medical device adhesives. It will be appreciated that any biocompatible adhesive is suitable for use with, in and/or as the backing layer. This layer may also be a nonwoven or porous film double coated with pressure sensitive adhesive.

Liquid backing formulations may be moved into the cavities by the same or similar methods as for the active agent casting solution. Where a liquid backing layer formulation is used, the solvent of the backing layer formulation is removed by a drying process. The drying conditions for drying the backing layer should be controlled so that the backing layer solvent can be removed effectively without affecting the stability of an active agent and/or to properly form (e.g. uniform) the backing layer. In one embodiment, the mold is placed into a compressed dry air (CDA) box under controlled air flow and then placed in an oven at about 5-50 °C.

The backing layer is typically first dried in a compressed dry air (CDA) box for a period of time with controlled air flow, e.g., from about 15 minutes to 2 hours, followed by drying in a convection oven, e.g., at a temperature ranging from 35°C to 80°C, for about 30 - 90 minutes. A backing substrate is then optionally placed on the backing or base layer. The backing substrate material can be, e.g., a breathable nonwoven pressure sensitive adhesive or an ultraviolet-cured adhesive in a polycarbonate film, although many types of materials can be used. Fig. 4 is an illustration of the casting method of forming microstructures having a drug-intip (DIT) and a backing layer. A liquid DIT solution is cast onto a mold having at least one cavity in the shape desired for the microstructures. The top surface of the mold is wiped to remove excess formulation. The liquid DIT is then dried under controlled conditions to remove the solvent resulting in a solid DIT layer in the bottom or distal end of the cavity. This dried DIT portion is the distal portion of the microstructure array. A backing layer is cast such that the remaining space in the cavity is filled and, optionally, a layer of backing layer formulation extends between the cavities. The backing layer is dried such that the resulting array has a backing layer with a plurality of microstructures extending at an angle from the backing layer. The backing layer with attached microstructures is demolded and preferably, but not always, undergoes a final drying step to form the microstructure array (MSA). It will be appreciated that the MSA may be demolded prior to undergoing the final drying step.

The microprojections with a backing layer may optionally be positioned on a base or substrate. The substrate may be in addition to or used in place of a backing layer. The microprojections or backing layer may be attached to the substrate by any suitable means. In one, non-limiting embodiment, the microstructures are attached to the substrate using an adhesive. Suitable adhesives include, but are not limited to, acrylic adhesives, acrylate adhesives, pressure sensitive adhesives, double-sided adhesive tape, double sided adhesive coated nonwoven or porous film, and UV curable adhesives. One exemplary double-sided tape is the #1513 double-coated medical tape available from 3M. Exemplary, but non-limiting, UV curable adhesives are the Dymax medical adhesives such as the 1187-M UV light-curable adhesive. It will be appreciated that any medical device adhesive known in the art would be suitable. In one embodiment, the substrate is a breathable nonwoven pressure sensitive adhesive. The substrate is placed on the backing layer where present or a proximal surface of the microprojections. The substrate is adhered or attached to the microprojections. In another embodiment, the substrate is a UV cured adhesive in a polycarbonate film. The UV adhesive is dispensed on the top of the backing layer or the proximal surface of the microprojections, covered with a polycarbonate (PC) film to spread the adhesive and cured using a UV Fusion system. In one embodiment a UV curing dose is about 1.6 J/cm$^2$. After the substrate is attached or adhered to the microprojections, the microprojection array is removed from the mold. It will be appreciated where the array includes a backing layer the substrate is attached or adhered to the backing layer as described above for the microstructures.

As described in Examples 2 and 4, a polymer matrix is cast onto a mold. Only the hydrophilic polymers as claimed are according to the invention. The mold is purged with $CO_2$ and excess formulation is removed from the mold top surface. The mold is pressurized and the formulation dried with a drying method to form the distal portions or ends of the microstructure arrays comprising the active agent(s). A polymer backing layer is cast onto the mold. The mold with the backing formulation is dried as described in Example 4 and above. In an optional embodiment, a backing substrate consisting of breathable, nonwoven layer and a pressure sensitive adhesive is placed on the backing layer as described in Example 5. In another embodiment, a UV adhesive is dispensed on the backing layer, covered with a polymer film such as a 5 mL PC film and the UV adhesive is cured using a UV Fusion system with a UV curing dose of 1.6 J/cm$^2$ to form a backing substrate.

Following removal from the mold, the microstructure array is typically die cut into appropriately sized sections, then may undergo a final drying step to remove residual moisture from the dried active agent-containing formulation and residual solvent from the backing layer. The final drying step may be conducted under vacuum (~6.66 Pa (~0.05 Torr)) at room temperature or higher, e.g., 35°C, for an extended period of several hours.

If desired, the microstructure arrays can then be packaged or sealed, either collectively or individually, preferably in airtight packaging. The packaged microstructure array(s) may also include a desiccant. A microstructure array as provided herein may also be provided as part of a kit, where the kit may also include an applicator device.

Characteristics of the Microstructure Arrays

The instant microstructure arrays comprise a dissolving biocompatible and water soluble matrix that stabilizes the active agent contained therein, in both liquid and in dried form (in terms of maintenance of chemical integrity and active agent potency) and additionally results in a microstructure array having good mechanical performance and good storage stability.

Exemplary microstructure arrays in accordance with the disclosure demonstrated advantageous active agent stability, both during manufacturing and upon storage. For instance, the active agent comprising biocompatible and water-soluble matrix, when dissolved in an aqueous buffer at an active agent concentration ranging from about 0.1% to about 7% by weight, is further characterized by stability of the active agent for at least 7 days at 5°C, as measured by one or more of maintenance of active agent particle size, chemical integrity, and active agent potency. Preferably, the liquid formulations used to prepare the microstructure array are sufficiently stable to maintain the integrity of the active agent during the manufacturing process. Exemplary methods of assessing stability of the formulations are described in Example 8. Moreover, microstructure arrays preferably possess good room temperature storage stability for an extended period of time (i.e., at least 1-3 months). See, e.g., Example 8. Finally, the immunogenic response resulting from the transdermal administration of an exemplary active agent via a microstructure array as provided herein is preferably at least as good as the response observed for intramuscular administration of a similar liquid active agent. Thus, the foregoing supports the advantageous features of the microstructure arrays, related formulations and methods provided herein.

Methods of Use

The methods, kits, microstructure arrays, and related devices and formulations described herein are used for transdermally administering an active agent to a human or veterinary subject.

The microstructure arrays described may be applied manually to the skin, e.g., by pressing the array into the skin. More preferably, an applicator is used to provide a mechanism for assisting in application of the microstructure array to and through the skin. A preferred type of applicator is one having a spring-loaded mechanism, to thereby drive the array into the skin by virtue of the energy stored in a spring. Suitable and illustrative applicators include those described in U.S. Publication No. 2011/0276027. For instance, an exemplary applicator will typically include a plunger or piston where the microstructure array is positioned on a distal end of the plunger, and an actuator (or actuating member) is actuated to thereby release the plunger. The plunger is typically held in a constrained or restrained position until released. Upon release of the plunger, the plunger then impacts the skin and thereby allows the microstructure array to pierce or rupture the skin surface. The remaining portion of the microstructure array may be removed from the plunger distal end automatically or manually.

Examples

The following examples are illustrative in nature and are in no way intended to be limiting. Efforts have been made to ensure accuracy with respect to numbers (e.g., amounts, temperature, etc.) but some errors and deviations should be accounted for. Unless indicated otherwise, parts are parts by weight, temperature is in °C, and pressure is at or near atmospheric.

Abbreviations

| | |
|---|---|
| API | Active pharmaceutical ingredient, |
| HPLC | High performance liquid chromatography |
| MSA | Microstructure array |
| SDS-PAGE | Sodium dodecyl sulfate polyacrylamide gel electrophoresis |
| SEC | Size exclusion chromatography |
| SPE | Skin penetration efficiency |
| TDS | Transdermal delivery system |
| DSL | Dynamic Light Scattering |
| IM | Intramuscular |

EXAMPLE 1

LIQUID FORMULATIONS CONTAINING ACTIVE AGENT

Vaccine active agent stock solutions or formulations are prepared by dissolving an antigen adjuvant, and polymer in an aqueous solution. Excipients including sugars, surfactants, and/or antioxidants are also added to the solvent. Liquid casting formulations (shown here excluding the vaccine antigen) are prepared by adding Dextran 70 (pharmaceutical grade, MW 70,000), sorbitol, and an aluminum salt ("alum") to an aqueous buffer and gently mixing the resulting solution by placing the container in a roller mixer (MediMix™) with a rolling speed <60 RPM for about 3 hours at room temperature. Two of the formulations further include adding an additional solvent such as isopropyl alcohol to the aqueous buffer. These two formulations (formulations 2 and 4) are according to the claims. The formulations 1 and 3, without at least one of the claimed co-solvents, are not according to the claims. Formulations are prepared as summarized in Table 1 below. Contact angle measurements are performed by dispensing about 5 μl drop of the respective formulation onto the flat portion of a silicone mold. images of the drop are captured and the contact angle of the liquid between the liquid drop and the silicone mold surface was measured using Image J.

**Table 1:** Liquid formulations

| Formulation Designation | Dextran70 (wt%) | Sorbitol (wt%) | Alum (wt%) | isopropyl alcohol (wt%) | Contact angle (degrees) |
|---|---|---|---|---|---|
| 1 | 14 | 5 | 1.1 | 0 | 110 |
| 2 | 14 | 5 | 1.1 | 10 | 79 |
| 3 | 14 | 5 | 3.3 | 0 | nm |
| 4 | 14 | 3 | 3.3 | 10 | nm |
| nm is not measured | | | | | |

EXAMPLE 2

CASTING MICROSTRUCTURE ARRAYS

Liquid casting formulations are prepared according to Example 1. Carbon dioxide is purged into the silicone mold prior to filling the liquid formulation into the mold cavities. After the $CO_2$ purge, liquid formulation gets dispensed onto the mold. Excess formulation is removed from the top surface of the mold. The mold is transferred to a petri dish and immediately placed into a pressurization chamber. Pressure (compressed dry air) is gradually increased to 206.84 kPa (30 psi) and maintained at 206.84 kPa (30 psi) for 90 seconds followed by a gradual decrease to zero kPa (zero psi). Pressurization after removal of excess formulation is believed to help in pushing the liquid formulation down into the cavities before drying process begins. Examples of microstructure arrays formed with and without pressurization after the initial drying step are shown in FIGS. 1A and 1B, respectively. After pressurization is completed, the liquid DIT in the mold is placed in an incubator oven at 32 °C for about 30 min for primary drying. The microstructure arrays formed using post wipe pressurization filled the cavities into the tips. Without post pressurization, the formulation was not filled into the very end of the cavity leaving air gaps, resulting in trunked needles as shown in Fig. 1B. Fig. 2A is an image of dried formulation in the mold prepared with pressurization after the formulation is wiped from the mold. Fig. 2B is an image of dried formulation in the mold where the formulation was not pressurized after excess formulation was wiped. The microstructures formed with pressurization are lower and deeper in the mold indicating the formulation was filled to the end (tip) of the mold cavity. As seen in Fig. 2B, the microstructures formed without pressurization are higher in the mold indicating the formulation did not fill the mold cavity.

EXAMPLE 3

LIQUID FORMULATIONS FOR BACKING LAYER

Different polymeric solutions may be used for casting a basement or backing layer for the microstructure arrays. Liquid formulations for a backing layer are prepared by dissolving one or more polymers in a solvent or solvent mixture at or about room temperature with a polymer concentration of about 10-40% by weight. An exemplary liquid formulations for casting a backing layer includes 30 wt% PLGA (75/25) dissolved in 70 wt% acetonitrile.

EXAMPLE 4

CASTING MICROSTRUCTURE ARRAYS WITH BACKING LAYER

Microstructures are prepared in accord with Example 2. A liquid backing layer formulation prepared in accord with Example 3 is dispensed on the mold. A thin film is cast by wiping the backing layer formulation. The mold is dried in a compressed dry air (CDA) box for about 30 minutes with controlled air flow. The mold is then dried in a convection oven at 45 °C for about 90 minutes to form the microstructure array (MSA) with a backing layer.

EXAMPLE 5

CASTING MICROSTRUCTURE ARRAYS WITH BACKING SUBSTRATE

A backing substrate may be used to connect the backing layer with an applicator device. Exemplary backing substrates include (i) a breathable non-woven pressure sensitive adhesive which is placed on the top of backing layer and (ii) an UV-curable adhesive cast on the backing layer and cured by UV, among others.
A microstructure array with a backing layer is prepared in accord with Example 4. A backing substrate consisting of a breathable nonwoven layer and pressure sensitive adhesive is placed on the backing layer. The MSA is removed from the mold and die cut into 1 or 2 $cm^2$ arrays. A final drying step is performed on the die cut MSA to completely remove any remnant moisture from the API casting formulations in the microstructure tips and residual solvent from the backing layer. This final drying is conducted under vacuum (~6.66 Pa (~0.05 Torr)) at 35 °C overnight. The MSAs are sealed individually in a Polyfoil pouch.

EXAMPLE 6

PREPARING ARRAY TOOL

A microstructure array tool with different geometries can be used to make the negative mold (generally using polydimethylsilicone). This mold is then used to fabricate a microstructure array (MSA) which replicates the geometry of the original tool. One exemplary tool used in these examples possesses a diamond shape with a microprojection height of 200 $\mu$m, a base width of 70 $\mu$m, and a projection-to-projection spacing of 200 $\mu$m. Figs. 2A-2B show an exemplary mold having diamond shaped cavities in use.

EXAMPLE 7

IN VITRO SKIN PENETRATION EFFICIENCY

Full-thickness pig skin is excised from the abdomen and then clipped and shaved to remove hair bristles. The MSAs prepared as described above are applied to shaved skin sites using an applicator to apply a force suitable to insert at least a portion of each microprojection into the skin and held by hand *in situ* for a period time ranging from about 5-15 minutes. Application sites are dye stained and photographed to visualize the microstructure array penetrations. Penetrations are quantified using a computer-based image analysis program. Skin penetration efficiency (SPE) is then calculated based on the theoretical number of microstructures expected for the MSA as follows:

$$\% \ SPE = 100 \times (\# \ Penetrations/\# \ Microstructures).$$

EXAMPLE 8

IN VIVO SKIN TOLERABILITY OF ALUM BY TRANSDERMAL ADMINISTRATION

While intramuscular and subcutaneous administration of alum-containing vaccines are generally well tolerated, it has been reported in literature that intradermal injection of alum-containing solutions can result in the formation of granulomas within the skin (Vogelbruch et al., Allergy, 2000, 55:883-887). An *in vivo* skin tolerability study was conducted in mini-pigs to assess local skin reaction to alum after intradermal administration by MSAs. Application sites were monitored over a 7 day period to assess skin irritation (erythema/edema) and nodule formation, if any. Alum MSA-treated sites were compared to intradermal syringe injections of liquid alum formulations. The amount of alum administered was the same for both methods. Two types of placebo MSAs (with dissolvable and non-dissolvable microstructures containing no alum) were also tested to assess whether formulation excipients and/or the mechanical act of penetrating the skin contributed to any irritation observed. Visual skin irritation assessments and histopathology assessments showed no apparent difference between the alum MSA-treated sites and the placebo MSA-treated sites during the 7 day period after applications. For all of the intradermal syringe injection sites, a small bump or nodule could be felt just under the skin beginning 4 to 7 days after injection. Histopathology assessments for the ID injection sites confirmed the presence of a foreign body reaction. No nodules or foreign body reactions were observed for the alum MSA-treated sites or for either of the placebo MSA-treated sites.

**Claims**

1.   A method of making a microstructure array, comprising:

   (i) providing an aqueous liquid formulation comprising a vaccine active agent, an insoluble particulate aluminum salt adjuvant, a hydrophilic polymer selected from the group consisting of polysaccharides, polyvinylpyrrolidones, polyvinyl alcohol, polyethylene glycol, copolymers of ethylene glycol and propylene glycol, and block copolymers of poly(lactic acid-co-glycolic acid) and polyethylene glycol, and at least one co-solvent selected from the group consisting of isopropyl alcohol, ethanol, methanol and butanol in an aqueous buffer;
   (ii) dispensing the liquid formulation from step (i) onto a mold having an array of microstructure cavities and filling the microstructure cavities to form a formulation-filled mold;
   (iii) removing excess liquid formulation from a top surface of the mold;
   (iv) drying the formulation-filled mold;
   (v) placing a backing layer on the dried mold from (iv), whereby the backing layer forms a base having an attachment point to the formulation dried in each of the microstructure cavities to provide a molded microstructure array, and
   (vi) removing the microstructure array from (v) from the mold.

2.   The method of claim 1, wherein the co-solvent is selected from isopropyl alcohol and ethanol.

3. The method of any one of claims 1 to 2, wherein the formulation comprises 5-20% of the co-solvent.

4. The method of any previous claim, further comprising purging the mold with a soluble gas prior to the dispensing step, preferably wherein the soluble gas is selected from $CO_2$ and $CH_4$.

5. The method of any one of claims 1 to 4, further comprising:

   applying pressure to the formulation filled mold after step (iii) or
   applying pressure to the formulation-filled mold after step (ii),

   preferably wherein applying pressure comprises applying pressure of at least 68.95 kPa (10 psi) above atmospheric or applying pressure of at least 206.84 kPa (30 psi) above atmospheric,
   more preferably wherein applying pressure comprises applying pressure for at least 5 seconds to 2 minutes.

6. The method of any previous claim, wherein drying the formulation-filled mold comprises drying the formulation-filled mold at 5-50° C for at least 30-60 minutes.

7. The method of any previous claim, further comprising:
   drying the backing layer formulation, preferably wherein drying the backing layer formulation comprises drying in an oven at 5-50° C.

8. The method of any previous claim, further comprising affixing a backing substrate to the backing layer, preferably wherein the backing substrate is selected from a pressure sensitive adhesive and a UV cured adhesive.

9. The method of any previous claim, further comprising:
   drying the microstructure array at 5-50° C for at least 12 hours, preferably wherein the drying is at 35° C.

10. The method of claim 9, wherein the drying is performed under vacuum, preferably wherein the drying is performed in a chamber having a partial pressure of water of 6.66 Pa (0.05 Torr).

11. The method of any previous claim, wherein the liquid formulation further comprises at least one of:

    a sugar selected from sorbitol, sucrose, trehalose, fructose, or dextrose,
    a surfactant selected from Polysorbate 20 or Polysorbate 80, or
    an antioxidant selected from methionine, cysteine, D-alpha tocopherol acetate, EDTA, or vitamin E.

12. The method of any previous claim, wherein the liquid formulation is a suspension.

13. A microstructure array obtained by the method of claim 1, comprising:

    an approximately planar base having a first surface and a second surface opposed thereto;
    a plurality of biodegradable microstructures extending outwardly from the base, each microstructure having an attachment point to the base and a distal portion to penetrate a subject's skin, wherein

        (i) the entire distal portion of each microstructure in the plurality of microstructures comprises at

            least one vaccine antigen active agent, and an insoluble particulate aluminum salt adjuvant in a biocompatible and water-soluble polymer matrix,
            the biocompatible and water-soluble polymer matrix comprising at least one structure forming polymer, wherein said structure forming polymer is a hydrophilic polymer selected from polysaccharides, polyvinyl pyrrolidone, polyvinyl alcohol, polyethylene glycol, copolymers of ethylene glycol and propylene glycol and block copolymers of poly(lactic acid-co-glycolic acid) and polyethylene glycol; and

        (ii) the base comprises a biocompatible non-water soluble polymer matrix,
        wherein the microstructures, upon penetration of the subject's skin, undergo dissolution to thereby deliver the antigen active agent and the particulate aluminum salt adjuvant.

14. The microstructure array of claim 13, wherein the vaccine antigen active agent is directed against at least one of adenovirus, anthrax, diphtheria, hepatitis, Haemophilus influenza a and/or b, human papillomavirus, influenza, Japanese encephalitis, Lyme disease, measles, meningococcal and pneumococcus infection, mumps, pertussis, polio, rabies, rotavirus, rubella, shingles, smallpox, tetanus, tuberculosis, typhoid, varicella, or yellow fever.

15. The microstructure array of any one of claims 13 to 14, wherein the particulate aluminum salt adjuvant is selected from aluminum hydroxide, aluminum potassium sulfate, and aluminum phosphate.

16. The microstructure array of any one of claims 13 to 15, wherein the plurality of microstructures further comprise at least one of:

a sugar selected from sorbitol, sucrose, trehalose, fructose, and dextrose,
a surfactant selected from Polysorbate 20 or Polysorbate 80, or
an antioxidant selected from methionine, cysteine, D-alpha tocopherol acetate, EDTA, or vitamin E.

17. The microstructure array of any one of claims 13 to 16, further comprising a backing substrate affixed to the planar base on an opposite side from the plurality of microstructures.

18. The microstructure array of any one of claims 13 to 17, wherein the microstructures have a diamond cross-section.

19. The microstructure array of any one of claims 13 to 18, wherein the microstructures have a height from tip to the backing layer of at least 50-500 $\mu$m, or a height of 100-300 $\mu$m, or a height of at least 200 $\mu$m.

## Patentansprüche

1. Verfahren zur Herstellung einer Mikrostrukturanordnung, umfassend:

(i) Bereitstellen einer wässrigen Flüssigformulierung, umfassend einen Impfstoffwirkstoff, ein unlösliches teilchenförmiges Aluminiumsalz-Adjuvans, ein hydrophiles Polymer, ausgewählt aus der Gruppe, bestehend aus Polysacchariden, Polyvinylpyrrolidonen, Polyvinylalkohol, Polyethylenglykol, Copolymeren von Ethylenglykol und Propylenglykol und Blockcopolymeren von Poly(milchsäure-co-Glykolsäure) und Polyethylenglykol, und mindestens einem Co-Lösungsmittel, ausgewählt aus der Gruppe, bestehend aus Isopropylalkohol, Ethanol, Methanol und Butanol in einem wässrigen Puffer;
(ii) Abgeben der flüssigen Formulierung aus Schritt (i) auf eine Form mit einer Anordnung von Mikrostrukturkavitäten und Befüllen der Mikrostrukturkavitäten zur Bildung einer mit der Formulierung befüllten Form;
(iii) Entfernen der überschüssigen flüssigen Formulierung von der oberen Oberfläche der Form;
(iv) Trocknen der mit der Formulierung befüllten Form;
(v) Aufbringen einer Stützschicht auf die getrocknete Form aus (iv), wobei die Stützschicht eine Basis mit einem Befestigungspunkt an der in jedem der Mikrostrukturkavitäten getrockneten Formulierung bildet, um eine geformte Mikrostrukturanordnung bereitzustellen, und
(vi) Entnahme der Mikrostrukturanordnung aus (v) aus der Form.

2. Verfahren nach Anspruch 1, wobei das Co-Lösungsmittel ausgewählt ist aus Isopropylalkohol und Ethanol.

3. Verfahren nach einem der Ansprüche 1 bis 2, wobei die Formulierung 5 bis 20 % des Co-Lösungsmittels umfasst.

4. Verfahren nach einem der vorstehenden Ansprüche, das ferner vor dem Abgabeschritt das Spülen der Form mit einem löslichen Gas umfasst, wobei das lösliche Gas vorzugsweise ausgewählt ist aus $CO_2$ und $CH_4$.

5. Verfahren nach einem der Ansprüche 1 bis 4, ferner umfassend:
Ausüben von Druck auf die mit der Formulierung befüllte Form nach Schritt (iii) oder Ausüben von Druck auf die mit der Formulierung befüllte Form nach Schritt (ii),
wobei das Ausüben von Druck vorzugsweise das Ausüben von Druck von mindestens 68,95 kPa (10 psi) über dem Atmosphärendruck oder das Ausüben von Druck von mindestens 206,84 kPa (30 psi) über dem Atmosphärendruck umfasst, wobei das Ausüben von Druck mehr bevorzugt das Ausüben von Druck für mindestens 5 Sekunden bis 2 Minuten umfasst.

6. Verfahren nach einem der vorstehenden Ansprüche, wobei das Trocknen der mit der Formulierung befüllten Form das Trocknen der mit der Formulierung befüllten Form bei 5 bis 50°C für mindestens 30 bis 60 Minuten umfasst.

7. Verfahren nach einem der vorstehenden Ansprüche, ferner umfassend:
Trocknen der Stützschichtformulierung, wobei das Trocknen der Stützschichtformulierung vorzugsweise das Trocknen in einem Ofen bei 5 bis 50°C umfasst.

8. Verfahren nach einem der vorstehenden Ansprüche, ferner umfassend das Anbringen eines Stützsubstrats an der Stützschicht, wobei das Stützsubstrat vorzugsweise ausgewählt ist aus einem druckempfindlichen Klebstoff und einem UV-gehärteten Klebstoff.

9. Verfahren nach einem der vorstehenden Ansprüche, ferner umfassend:
Trocknen der Mikrostrukturanordnung mindestens 12 Stunden lang bei 5 bis 50° C, wobei die Trocknung vorzugsweise bei 35° C erfolgt.

10. Verfahren nach Anspruch 9, wobei die Trocknung unter Vakuum durchgeführt wird, wobei die Trocknung vorzugsweise in einer Kammer mit einem Wasserpartialdruck von 6,66 Pa (0,05 Torr) durchgeführt wird.

11. Verfahren nach einem der vorstehenden Ansprüche, wobei die flüssige Formulierung ferner mindestens eines der Folgenden umfasst:

einen Zucker, ausgewählt aus Sorbit, Saccharose, Trehalose, Fructose oder Dextrose,
ein Tensid, ausgewählt aus Polysorbat 20 oder Polysorbat 80, oder
ein Antioxidans, ausgewählt aus Methionin, Cystein, D-alpha-Tocopherolacetat, EDTA oder Vitamin E.

12. Verfahren nach einem der vorstehenden Ansprüche, wobei es sich bei der flüssigen Formulierung um eine Suspension handelt.

13. Mikrostrukturanordnung, die durch das Verfahren nach Anspruch 1 erzeugt wird, umfassend:

eine annähernd ebene Basis mit einer ersten Oberfläche und einer dieser gegenüberliegenden zweiten Oberfläche;
eine Vielzahl von biologisch abbaubaren Mikrostrukturen, die von der Basis nach außen verlaufen, wobei jede Mikrostruktur einen Befestigungspunkt an der Basis und einen distalen Abschnitt zum Durchstoßen der Haut einer Person aufweist, wobei

(i) der gesamte distale Abschnitt jeder Mikrostruktur in der Vielzahl von Mikrostrukturen mindestens einen Impfstoff-Antigen-Wirkstoff und ein unlösliches teilchenförmiges Aluminiumsalz-Adjuvans in einer biokompatiblen und wasserlöslichen Polymermatrix umfasst,
wobei die biokompatible und wasserlösliche Polymermatrix mindestens ein strukturbildendes Polymer umfasst, wobei es sich bei dem strukturbildenden Polymer um ein hydrophiles Polymer handelt, ausgewählt aus Polysacchariden, Polyvinylpyrrolidon, Polyvinylalkohol, Polyethylenglykol, Copolymeren von Ethylenglykol und Propylenglykol und Blockcopolymeren von Poly(milchsäureco-Glykolsäure) und Polyethylenglykol,
und
(ii) wobei die Basis eine biokompatible, nicht wasserlösliche Polymermatrix umfasst, wobei sich die Mikrostrukturen nach dem Durchstoßen der Haut der Person auflösen, um dadurch den Antigenwirkstoff und das teilchenförmige Aluminiumsalz-Adjuvans freizusetzen.

14. Mikrostrukturanordnung nach Anspruch 13, wobei der Impfstoff-Antigen-Wirkstoff gegen mindestens eines der folgenden Viren gerichtet ist: Adenovirus, Anthrax, Diphtherie, Hepatitis, Haemophilus influenza a und/oder b, humanes Papillomavirus, Influenza, Japanische Enzephalitis, Borreliose, Masern, Meningokokken- und Pneumokokkeninfektionen, Mumps, Keuchhusten, Polio, Tollwut, Rotavirus, Röteln, Gürtelrose, Pocken, Tetanus, Tuberkulose, Typhus, Windpocken oder Gelbfieber.

15. Mikrostrukturanordnung nach einem der Ansprüche 13 bis 14, wobei das teilchenförmige Aluminiumsalz-Adjuvans ausgewählt ist aus Aluminiumhydroxid, Aluminiumkaliumsulfat und Aluminiumphosphat.

16. Mikrostrukturanordnung nach einem der Ansprüche 13 bis 15, wobei die Vielzahl von Mikrostrukturen ferner mindestens eines der Folgenden umfasst:

einen Zucker, ausgewählt aus Sorbit, Saccharose, Trehalose, Fructose und Dextrose,
ein Tensid, ausgewählt aus Polysorbat 20 oder Polysorbat 80, oder
ein Antioxidans, ausgewählt aus Methionin, Cystein, D-alpha-Tocopherolacetat, EDTA oder Vitamin E.

**17.** Mikrostrukturanordnung nach einem der Ansprüche 13 bis 16, ferner umfassend ein Stützsubstrat, das an der ebenen Basis auf einer der Vielzahl von Mikrostrukturen gegenüberliegenden Seite befestigt ist.

**18.** Mikrostrukturanordnung nach einem der Ansprüche 13 bis 17, wobei die Mikrostrukturen einen diamantförmigen Querschnitt aufweisen.

**19.** Mikrostrukturanordnung nach einem der Ansprüche 13 bis 18, wobei die Mikrostrukturen eine Höhe von der Spitze bis zur Stützschicht von mindestens 50 bis 500 $\mu$m oder eine Höhe von 100 bis 300 $\mu$m oder eine Höhe von mindestens 200 $\mu$m aufweisen.

**Revendications**

**1.** Procédé de fabrication d'un réseau de microstructures, comprenant :

(i) la mise à disposition d'une formulation liquide aqueuse comprenant un agent actif de vaccin, un adjuvant de sel d'aluminium particulaire insoluble, un polymère hydrophile choisi dans le groupe constitué par des polysaccharides, des polyvinylpyrrolidones, un poly(alcool vinylique), un polyéthylène glycol, des copolymères d'éthylène glycol et de propylèneglycol, et des copolymères à blocs de poly(acide lactique-co-acide glycolique) et de polyéthylène glycol, et au moins un cosolvant choisi dans le groupe constitué par l'alcool isopropylique, l'éthanol, le méthanol et le butanol dans un tampon aqueux ;
(ii) la distribution de la formulation liquide de l'étape (i) sur un moule possédant un réseau de cavités de microstructures et le remplissage des cavités de microstructures pour former un moule rempli par la formulation ;
(iii) l'élimination de l'excès de formulation liquide d'une surface supérieure du moule ;
(iv) le séchage du moule rempli par la formulation ;
(v) le placement d'une couche de support sur le moule séché de (iv), moyennant quoi la couche de support forme une base possédant un point de fixation à la formulation séchée dans chacune des cavités de microstructures pour fournir un réseau de microstructures moulé, et
(vi) l'élimination du réseau de microstructures de (v) du moule.

**2.** Procédé selon la revendication 1, le cosolvant étant choisi parmi l'alcool isopropylique et l'éthanol.

**3.** Procédé selon l'une quelconque des revendications 1 à 2, la formulation comprenant 5 à 20 % du cosolvant.

**4.** Procédé selon une quelconque revendication précédente, comprenant en outre une purge du moule avec un gaz soluble avant l'étape de distribution, préférablement le gaz soluble étant choisi parmi $CO_2$ et $CH_4$.

**5.** Procédé selon l'une quelconque des revendications 1 à 4, comprenant en outre :

l'application d'une pression sur le moule rempli par la formulation après l'étape (iii) ou
l'application d'une pression sur le moule rempli par la formulation après l'étape (ii),
préférablement, l'application d'une pression comprenant l'application d'une pression d'au moins 68,95 kPa (10 psi) au-dessus de la pression atmosphérique ou l'application d'une pression d'au moins 206,84 kPa (30 psi) au-dessus de la pression atmosphérique,
plus préférablement, l'application d'une pression comprenant l'application d'une pression pendant au moins 5 secondes à 2 minutes.

**6.** Procédé selon une quelconque revendication précédente, le séchage du moule rempli par la formulation comprenant le séchage du moule rempli par la formulation à une température de 5 à 50 °C pendant au moins 30 à 60 minutes.

**7.** Procédé selon une quelconque revendication, comprenant en outre :
le séchage de la formulation de couche de support, préférablement le séchage de la formulation de couche de support comprenant le séchage dans un four à une température de 5 à 50 °C.

8. Procédé selon une quelconque revendication précédente, comprenant en outre la fixation d'un substrat de support sur la couche de support, préférablement, le substrat de support étant choisi parmi un adhésif sensible à la pression et un adhésif durci par des UV.

9. Procédé selon une quelconque revendication précédente, comprenant en outre :
le séchage du réseau de microstructures à une température de 5 à 50 °C pendant au moins 12 heures, préférablement, le séchage étant à une température de 35 °C.

10. Procédé selon la revendication 9, le séchage étant réalisé sous vide, préférablement, le séchage étant réalisé dans un compartiment possédant une pression partielle d'eau de 6,66 Pa (0,05 Torr).

11. Procédé selon une quelconque revendication précédente, la formulation liquide comprenant en outre au moins l'un parmi :

un sucre choisi parmi le sorbitol, le saccharose, le tréhalose, le fructose ou le dextrose,
un tensioactif choisi parmi le polysorbate 20 ou le polysorbate 80, ou
un antioxydant choisi parmi la méthionine, la cystéine, l'acétate de D-alpha-tocophérol, l'EDTA ou la vitamine E.

12. Procédé selon une quelconque revendication précédente, la formulation liquide étant une suspension.

13. Réseau de microstructures obtenu par le procédé selon la revendication 1, comprenant :

une base approximativement plane possédant une première surface et une deuxième surface opposée à celle-ci ;
une pluralité de microstructures biodégradables s'étendant vers l'extérieur à partir de la base,
chaque microstructure possédant un point de fixation à la base et une partie distale pour pénétrer une peau d'un sujet,

(i) la partie distale entière de chaque microstructure dans la pluralité de microstructures comprenant au moins un agent actif d'antigène de vaccin et un adjuvant de sel d'aluminium particulaire insoluble dans une matrice polymérique biocompatible et soluble dans l'eau,
la matrice polymérique biocompatible et soluble dans l'eau comprenant au moins un polymère de formation de structure, ledit polymère de formation de structure étant un polymère hydrophile choisi parmi des polysaccharides, des polyvinylpyrrolidones, un poly(alcool vinylique), un polyéthylène glycol, des copolymères d'éthylène glycol et de propylèneglycol, et des copolymères à blocs de poly(acide lactique-co-acide glycolique) et de polyéthylène glycol ; et
(ii) la base comprenant une matrice polymérique non soluble dans l'eau biocompatible,
les microstructures, lors d'une pénétration de la peau d'un sujet, subissant une dissolution pour ainsi délivrer l'agent actif d'antigène et l'adjuvant de sel d'aluminium particulaire.

14. Réseau de microstructures selon la revendication 13, l'agent actif d'antigène vaccinal étant dirigé contre au moins l'un parmi un adénovirus, l'anthrax, la diphtérie, l'hépatite, la grippe hémophile a et/ou b, le papillomavirus humain, la grippe, l'encéphalite japonaise, la maladie de Lyme, la rougeole, une infection à méningocoques et pneumocoques, les oreillons, la coqueluche, la polio, la rage, un rotavirus, la rubéole, le zona, la variole, le tétanos, la tuberculose, la typhoïde, la varicelle ou la fièvre jaune.

15. Réseau de microstructures selon l'une quelconque des revendications 13 à 14, l'adjuvant de sel d'aluminium particulaire étant choisi parmi l'hydroxyde d'aluminium, le sulfate d'aluminium potassium et le phosphate d'aluminium.

16. Réseau de microstructures selon l'une quelconque des revendications 13 à 15, la pluralité de microstructures comprenant en outre au moins l'un parmi :

un sucre choisi parmi le sorbitol, le saccharose, le tréhalose, le fructose ou le dextrose,
un tensioactif choisi parmi le polysorbate 20 ou le polysorbate 80, ou
un antioxydant choisi parmi la méthionine, la cystéine, l'acétate de D-alpha-tocophérol, l'EDTA ou la vitamine E.

**17.** Réseau de microstructures selon l'une quelconque des revendications 13 à 16, comprenant en outre un substrat de support fixé à la base plane sur un côté opposé de la pluralité de microstructures.

**18.** Réseau de microstructures selon l'une quelconque des revendications 13 à 17, les microstructures possédant une section transversale en diamant.

**19.** Réseau de microstructures selon l'une quelconque des revendications 13 à 18, les microstructures possédant une hauteur du sommet à la couche de support d'au moins 50 à 500 pm, ou une hauteur de 100 à 300 pm, ou une hauteur d'au moins 200 pm.

FIG. 1A

FIG. 1B

FIG. 2A

FIG. 2B

**FIG. 3**

**FIG. 4**

FIG. 5A

FIG. 5B

FIG. 5C

**FIG. 5D**

**FIG. 5E**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62044051
- US 6451240 B
- US 6945952 B
- US 20020082543 A
- US 20050197308 A
- US 20090155330 A
- US 2009035446 A1
- US 6219574 B
- US 6652478 B
- US 20080269685 A
- US 20110006458 A
- US 20110276028 A
- US 20040087992 A
- US 61745513
- US 5900252 A
- US 7785301 B
- US 20110276027 A

### Non-patent literature cited in the description

- **PARK et al.** *J. of Controlled Release,* 2005, vol. 104, 51-66
- **PRAUSNITZ et al.** *Curr Top Microbiol Immunol,* 2009, vol. 333, 369-393
- **MUNKS et al.** *Blood,* 2010, vol. 116 (24), 5191-5199
- **A.L. LEHNINGER.** Biochemistry. Worth Publishers, Inc,
- **MORRISON ; BOYD.** Organic Chemistry. Allyn and Bacon, Inc,
- **J. MARCH.** Advanced Organic Chemistry. McGraw Hill
- Remington: The Science and Practice of Pharmacy
- **J. GRIFFITH HARDMAN ; L. L. LIMBIRD ; A. GILMAN.** Goodman & Gilman The Pharmacological Basis of Therapeutics
- **WHITE et al.** *J Exp Med,* 1955, vol. 102 (1), 73-82
- **AVCIN et al.** *Acta Dermatoven APA,* 2007, vol. 17 (4), 182-184
- **PITTMAN.** *Vaccine,* 2002, S48-S50
- Remington's Pharmaceutical Sciences. 1990
- **VOGELBRUCH et al.** *Allergy,* 2000, vol. 55, 883-887